# EUROPEAN PATENT APPLICATION

(11) **EP 1 918 711 A2**
(43) Date of publication of application: **07.05.2008**
(21) Application number: 07254302.8
(22) Date of filing: 30.10.2007
(51) Int. Cl.: G01N 33/574, C12Q 1/68

(54) **Prostate cancer field effect analysis methods and kits**

(30) Priority: 31.10.2006 US 855640 P
(71) Applicant: Veridex, LLC, Warren, NJ 07059 (US)
(72) Inventor: Mehrotra, Jyoti, Bridgewater, NJ 08807 (US); Varde, Shobha, Jacksonville, FL 32258 (US); Gray, Karen, Warren, NJ 07059 (US); Vargo, Janet, Warren, NJ 07059 (US); Mazumder, Abhijit, Basking Ridge, NJ 07920 (US)
(74) Representative: Mercer, Christopher Paul

(57) **Abstract**

Methods and kits for characterizing prostate cancer include detecting the methylation status of various genes.

## Description

### CROSS REFERENCE TO RELATED APPLICATION

This application claims the benefit of U.S. Provisional Patent Application Serial Number 60/855640 filed October 31, 2006.

### BACKGROUND OF THE INVENTION

This invention relates to the interrogation of methylated genes present in regions near a tumor such as in surgical margins.

The premise of field cancerization, also referred to as the field effect, was first proposed in the 1950s to explain the occurrence of multiple primary tumors and local recurrence. The concept was based on the observations that cancer developed in multifocal areas and that abnormal tissue surrounded the tumor, suggesting that neoplastic or preneoplastic cells existed in a histologically-normal field proximal to the tumor.

The standard technique for assessing the spread of a tumor is surgical resection of a primary tumor followed by careful review using light microscopy of surgical margins and other tissue. Under existing procedure, the adjacent tissue is stained by standard techniques and assessed under light microscopy for the presence of tumor cells. Accurate therapeutic staging assesses the extent of tumor spread locally as well as the presence of regional metastases in more distant sites, such as lymph nodes. Histopathologic assessment provides prognostic indicators that help determine the probability of survival.

The standard histopathologic methodology based upon visual observation and morphologic assessment under light microscopy of adjacent tissue and regional lymph nodes is known to have limitations. Methods which can produce information that is more capable of determining spread of the disease at an earlier stage and a more accurate indication of the extent of tumor metastases into adjacent and regional tissues are desirable. The present invention provides such a method based on detecting methylation markers in samples.

In higher order eukaryotes DNA is methylated only at cytosines located 5' to guanosine in the CpG dinucleotide. This modification has important regulatory effects on gene expression, especially when it involves CpG rich areas (CpG islands) located in gene promoter regions. Abberant methylation of normally unmethylated CpG islands is a frequent event in immortalized and transformed cells and has been associated with transcriptional inactivation of certain tumor suppressor genes or genes otherwise associated with the amelioration of certain human cancers. Detecting methylation events such as these can provide diagnostic and/or prognostic information where histopathologic information is not clear or altogether helpful or it can add to the utility of histopathologic information.

### SUMMARY OF THE INVENTION

One aspect of the invention is a method for characterizing prostate cancer based on detecting a methylation Marker that is detectable in the adjacent histopathologic surgical margins that appear normal when examined by standard histological techniques.

The present invention provides a method that can be used as an adjunct to cytopathology, to screen high-risk populations and to monitor high-risk patients undergoing chemoprevention or chemotherapy.

Another aspect of the invention is a kit for characterizing prostate cancer. The kit includes reagents for amplifying and detecting methylation Markers and contains instructions for their use on prostate cancer patients using surgical margin samples.

In another aspect of the invention, a patient is treated consistent with the manner in which those with aggressive or indolent prostate cancers are treated depending upon the presence or absence of methylation Markers in tumor margins.

In yet another aspect of the invention, hypermethylation of one or more genes from the following group is detected in a surgical margin sample: APC, RASSF1A, and RAR β2.

A patient's condition with respect prostate cancer is characterized based on such detection. Detection of hypermethylation is an indicator of poor prognosis for metastasis or aggressiveness of the disease. The prognosis is most preferably made in conjunction with other indicators.

In another aspect of the invention, methylation status is determined via quantitative real time PCR.

### BRIEF DESCRIPTION OF THE DRAWINGS

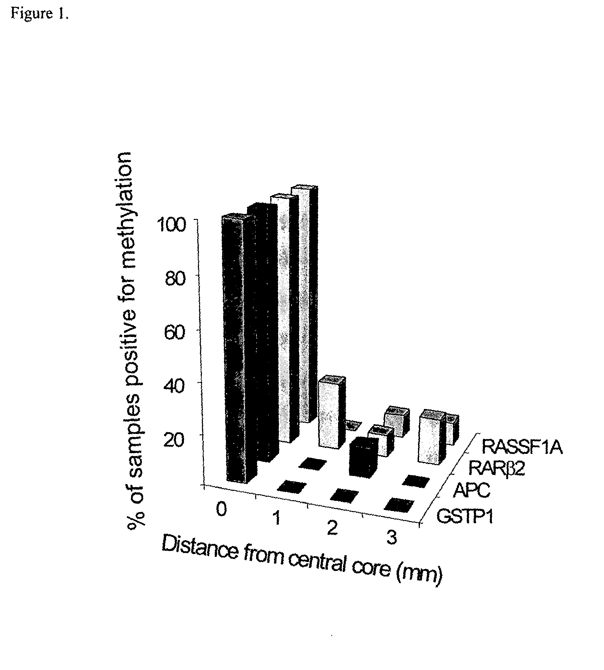

Figure 1 is a graphical representation showing the number of samples showing a field effect as a function of the distance from the central core. For each marker, the number of samples exhibiting a methylation ratio (of any magnitude) at a particular distance was divided by the total number of prostatectomy samples which had a methylation ratio for the central, malignant core (C₀) and which had no cancer in the subsequent cores.

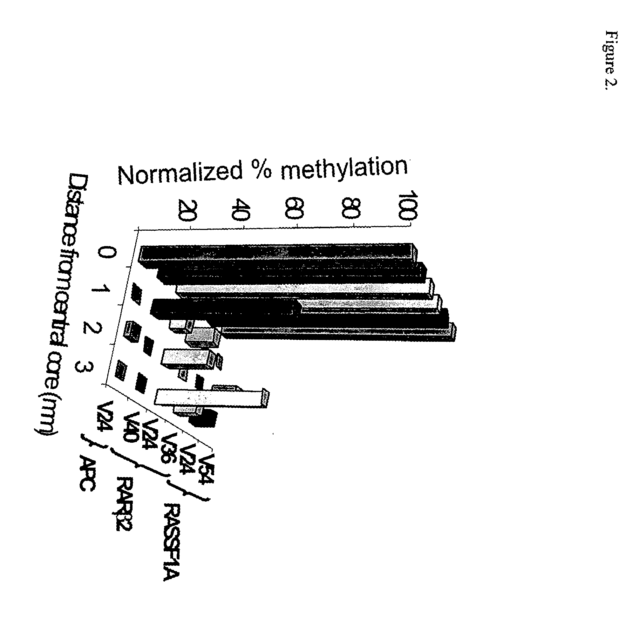

Figure 2 is a graphical representation of variety in the field effect. The normalized percent methylation was calculated by dividing the methylation ratio obtained with a particular core by the methylation ratio obtained with the corresponding C₀ core and multiplying by 100. For each of the samples that exhibited a methylation ratio in Example 3, the normalized percent methylation was tracked as a function of distance from C₀ for APC (front row), RARβ2 (second, third, and fourth rows), and RASSF1A (last two rows).

### DETAILED DESCRIPTION OF THE INVENTION

The present invention relates to a method of detecting mutant nucleotide sequence present in a histopathologic tissue sample external to a primary neoplasm, such as a tumor margin specimen. The mutations in this case are hypermethylations of the sequences of certain Markers. The Markers are nucleic acids that correspond to genes whose methylation status correlates with prostate cancer status.

The term "neoplastic" refers to being directly or indirectly related to or causing a neoplasm. The term "tumor margin" refers to the tissue surrounding a discernible tumor. In the case of surgical removal of a solid tumor, the tumor margin is the tissue cut away with the discernible tumor that usually appears to be normal to the naked eye. More particularly, "margin" refers to the edge, border or boundary of a tumor. The margin generally extends from about 1 mm to about 4 mm from the primary tumor but can be greater depending upon the size of the primary solid tumor.

A nucleic acid "corresponds" to a gene whose methylation status correlates with prostate cancer when the methylation status of such a gene provides information about prostate cancer and the sequence is a coding portion of the gene or its complement, a representative portion of the gene or its complement, a promoter or regulatory sequence for the gene or its complement, a sequence that indicates the presence of the gene or its complement, or the full length sequence of the gene or its complement. Such nucleic acids are referred to as Markers in this specification. Markers correspond to the following genes: HIN (Seq ID No. 58), RASSF1A (Seq. ID. No. 69), APC (Promoter-Seq. ID. No. 64, Gene= Seq. ID. No. 65), RAR β2 (Seq. ID. No. 62). Other sequences of interest include constitutive genes useful as assay controls such as beta-Actin (Seq. ID. No.60 and 61) and PTGS2 (Promoter= Seq. ID. No.66, Gene= Seq. ID. No. 67). GSTP1 (Seq. ID. No. 59) is a gene of interest.

Assays for detecting hypermethylation include such techniques as MSP and restriction endonuclease analysis. The promoter region is a particularly noteworthy target for detecting such hypermethylation analysis.

The invention includes determining the methylation status of certain regions of the Markers in a tissue or other biological sample of a subject in which the DNA associated with prostate cancer is amplified and detected. Since a decreased level of the protein encoded by the gene to which the Marker corresponds (i.e., less transcription) is often the result of hypermethylation of a particular region such as the promoter, it is desirable to determine whether such regions are hypermethylated. Hypermethylated regions are those that are methylated to a statistically significant greater degree in samples from diseased tissue (or tissue that is effected such that it is reflective of a poor prognosis) as compared to normal tissue. Hypermethylation as detected by the use of Markers on tumor margin samples is indicative of a neoplasm or likely neoplasm development outside the primary tumor. It is also indicative of a more aggressive and potentially or actually more metastasized cancer. Accordingly, it delineates which patients are treated more aggressively and which patients can be treated more conservatively such as with watchful waiting.

Oligonucleotide primers based on certain portions of the Marker sequence are particularly useful for amplifying DNA by techniques such as PCR. Some of the primers/probes or reporter reagents of the invention are used to detect methylation of expression control sequences of the Marker genes. These are nucleic acid sequences that regulate the transcription and, in some cases, translation of the nucleic acid sequence. Thus, expression control sequences can include sequences involved with promoters, enhancers, transcription terminators, start codons (i.e., ATG), splicing signals for introns, maintenance of the correct reading frame of that gene to permit proper translation of the mRNA, and stop codons.

One method of the invention includes contacting a target cell containing a Marker with a reagent that binds to the nucleic acid. The target cell component is a nucleic acid such as DNA or RNA. The reagents can include probes and primers such as PCR or MSP primers or other molecules configured to amplify and detect the target sequence. For example, the reagents can include priming sequences combined with or bonded to their own reporter segments such as those referred to as Scorpion reagents or Scorpion reporters and described in US Patents 6,326,145 and 6,270,967 to Whitcombe *et. al.* (incorporated herein by reference in their entirety). Though they are not the same, the terms "primers" and "priming sequences" may be used in this specification to refer to molecules or portions of molecules that prime the amplification of nucleic acid sequences.

One sensitive method of detecting methylation patterns involves combining the use of methylation-sensitive enzymes and the polymerase chain reaction (PCR). After digestion of DNA with the enzyme, PCR will amplify from primers flanking the restriction site only if DNA cleavage was prevented by methylation.

The method of the invention can also include contacting a nucleic acid-containing specimen with an agent that modifies unmethylated cytosine; amplifying the CpG-containing nucleic acid in the specimen by means of CpG-specific oligonucleotide primers; and detecting the methylated nucleic acid. The preferred modification is the conversion of unmethylated cytosines to another nucleotide that will distinguish the unmethylated from the methylated cytosine. Preferably, the agent modifies unmethylated cytosine to uracil and is sodium bisulfite, however, other agents that modify unmethylated cytosine, but not methylated cytosine can also be used. Sodium bisulfite (NaHSO₃) modification is most preferred and reacts readily with the 5,6-double bond of cytosine, but poorly with methylated cytosine. Cytosine reacts with the bisulfite ion to form a sulfonated cytosine reaction intermediate susceptible to deamination, giving rise to a sulfonated uracil. The sulfonate group can be removed under alkaline conditions, resulting in the formation of uracil. Uracil is recognized as a thymine by Taq polymerase and therefore upon PCR, the resultant product contains cytosine only at the position where 5-methylcytosine occurs in the starting template. Scorpion reporters and reagents and other detection systems similarly distinguish modified from unmodified species treated in this manner.

The primers used in the invention for amplification of a CpG-containing nucleic acid in the specimen, after modification (e.g., with bisulfite), specifically distinguish between untreated DNA, methylated, and non-methylated DNA. In methylation specific PCR (MSPCR), primers or priming sequences for the non-methylated DNA preferably have a T in the 3' CG pair to distinguish it from the C retained in methylated DNA, and the complement is designed for the antisense primer. MSP primers or priming sequences for non-methylated DNA usually contain relatively few Cs or Gs in the sequence since the Cs will be absent in the sense primer and the Gs absent in the antisense primer (C becomes modified to U (uracil) which is amplified as T (thymidine) in the amplification product).

The primers of the invention are oligonucleotides of sufficient length and appropriate sequence so as to provide specific initiation of polymerization on a significant number of nucleic acids in the polymorphic locus. When exposed to appropriate probes or reporters, the sequences that are amplified reveal methylation status and thus diagnostic information.

Preferred primers are made up of eight or more deoxyribonucleotides or ribonucleotides capable of initiating synthesis of a primer extension product, which is substantially complementary to a polymorphic locus strand. Environmental conditions conducive to synthesis include the presence of nucleoside triphosphates and an agent for polymerization, such as DNA polymerase, and a suitable temperature and pH. The priming segment of the primer or priming sequence is preferably single stranded for maximum efficiency in amplification, but may be double stranded. If double stranded, the primer is first treated to separate its strands before being used to prepare extension products. The primer must be sufficiently long to prime the synthesis of extension products in the presence of the inducing agent for polymerization. The exact length of primer will depend on factors such as temperature, buffer, and nucleotide composition. The oligonucleotide primers most preferably contain about 12-20 nucleotides although they may contain more or fewer nucleotides, preferably according to well-known design guidelines or rules.

Primers are designed to be substantially complementary to each strand of the genomic locus to be amplified and include the appropriate G or C nucleotides as discussed above. This means that the primers must be sufficiently complementary to hybridize with their respective strands under conditions that allow the agent for polymerization to perform. In other words, the primers should be sufficient complementary with the 5' and 3' flanking sequence(s) to hybridize and permit amplification of the genomic locus.

The primers are employed in the amplification process. That is, reactions (preferably, an enzymatic chain reaction) that produce greater quantities of target locus relative to the number of reaction steps involved. In a most preferred embodiment, the reaction produces exponentially greater quantities of the target locus. Reactions such as these include the PCR reaction. Typically, one primer is complementary to the negative (-) strand of the locus and the other is complementary to the positive (+) strand. Annealing the primers to denatured nucleic acid followed by extension with an enzyme, such as the large fragment of DNA Polymerase I (Klenow) and nucleotides, results in newly synthesized + and - strands containing the target locus sequence. The product of the chain reaction is a discrete nucleic acid duplex with termini corresponding to the ends of the specific primers employed.

The primers may be prepared using any suitable method, such as conventional phosphotriester and phosphodiester methods including automated methods. In one such automated embodiment, diethylphosphoramidites are used as starting materials and may be synthesized as described by Beaucage, et at. (Tetrahedron Letters, 22:1859-1862, 1981). A method for synthesizing oligonucleotides on a modified solid support is described in U.S. Pat. No. 4,458,066.

Any nucleic acid specimen obtained from surgical margin samples, in purified or non-purified form, can be utilized as the starting nucleic acid or acids of this invention, provided it contains, or is suspected of containing, the specific nucleic acid sequence containing the target locus (e.g., CpG). Thus, the process may employ, for example, DNA or RNA, including messenger RNA. The DNA or RNA may be single stranded or double stranded. In the event that RNA is to be used as a template, enzymes, and/or conditions optimal for reverse transcribing the template to DNA would be utilized. In addition, a DNA-RNA hybrid containing one strand of each may be utilized. A mixture of nucleic acids may also be employed, or the nucleic acids produced in a previous amplification reaction herein, using the same or different primers may be so utilized. The specific nucleic acid sequence to be amplified, i.e., the target locus, may be a fraction of a larger molecule or can be present initially as a discrete molecule so that the specific sequence constitutes the entire nucleic acid. The nucleic acid-containing specimen may be extracted by a variety of techniques such as that described by Maniatis, et al. (Molecular Cloning: A Laboratory Manual, Cold Spring Harbor, N.Y., pp 280, 281, 1982).

If the extracted sample is impure, it may be treated before amplification with an amount of a reagent effective to open the cells, fluids, tissues, or animal cell membranes of the sample, and to expose and/or separate the strand(s) of the nucleic acid(s). This lysing and nucleic acid denaturing step to expose and separate the strands will allow amplification to occur much more readily.

Where the target nucleic acid sequence of the sample contains two strands, it is necessary to separate the strands of the nucleic acid before it can be used as the template. Strand separation can be effected either as a separate step or simultaneously with the synthesis of the primer extension products. This strand separation can be accomplished using various suitable denaturing conditions, including physical, chemical or enzymatic means. One physical method of separating nucleic acid strands involves heating the nucleic acid until it is denatured. Typical heat denaturation may involve temperatures ranging from about 80 to 105°C for up to 10 minutes. Strand separation may also be induced by an enzyme from the class of enzymes known as helicases or by the enzyme RecA, which has helicase activity, and in the presence of riboATP, is known to denature DNA. Reaction conditions that are suitable for strand separation of nucleic acids using helicases are described by Kuhn Hoffmann-Berling (CSH-Quantitative Biology, 43:63, 1978). Techniques for using RecA are reviewed in C. Radding (Ann. Rev. Genetics, 16:405-437, 1982). Refinements of these techniques are now also well known.

When complementary strands of nucleic acid or acids are separated, regardless of whether the nucleic acid was originally double or single stranded, the separated strands are ready to be used as a template for the synthesis of additional nucleic acid strands. This synthesis is performed under conditions allowing hybridization of primers to templates to occur. Generally synthesis occurs in a buffered aqueous solution, preferably at a pH of 7-9, most preferably about 8. A molar excess (for genomic nucleic acid, usually about 10⁸:1, primer:template) of the two oligonucleotide primers is preferably added to the buffer containing the separated template strands. The amount of complementary strand may not be known if the process of the invention is used for diagnostic applications, so the amount of primer relative to the amount of complementary strand cannot always be determined with certainty. As a practical matter, however, the amount of primer added will generally be in molar excess over the amount of complementary strand (template) when the sequence to be amplified is contained in a mixture of complicated long-chain nucleic acid strands. A large molar excess is preferred to improve the efficiency of the process.

The deoxyribonucleoside triphosphates dATP, dCTP, dGTP, and dTTP are added to the synthesis mixture, either separately or together with the primers, in adequate amounts and the resulting solution is heated to about 90-100°C for up to 10 minutes, preferably from 1 to 4 minutes. After this heating period, the solution is allowed to cool to room temperature, which is preferable for the primer hybridization. To the cooled mixture is added an appropriate agent for effecting the primer extension reaction (the "agent for polymerization"), and the reaction is allowed to occur under conditions known in the art. The agent for polymerization may also be added together with the other reagents if it is heat stable. This synthesis (or amplification) reaction may occur at room temperature up to a temperature at which the agent for polymerization no longer functions.

The agent for polymerization may be any compound or system that will function to accomplish the synthesis of primer extension products, preferably enzymes. Suitable enzymes for this purpose include, for example, E. coli DNA polymerase 1, Klenow fragment ofE. coli DNA polymerase I, T4 DNA polymerase, other available DNA polymerases, polymerase mutants, reverse transcriptase, and other enzymes, including heat-stable enzymes (e.g., those enzymes which perform primer extension after being subjected to temperatures sufficiently elevated to cause denaturating). A preferred agent is Taq polymerase. Suitable enzymes will facilitate combination of the nucleotides in the proper manner to form the primer extension products complementary to each locus nucleic acid strand. Generally, the synthesis will be initiated at the 3' end of each primer and proceed in the 5' direction along the template strand, until synthesis terminates, producing molecules of different lengths. There may be agents for polymerization, however, which initiate synthesis at the 5' end and proceed in the other direction, using the same process as described above.

Most preferably, the method of amplifying is by PCR. Alternative methods of amplification can also be employed as long as the methylated and non-methylated loci amplified by PCR using the primers of the invention is similarly amplified by the alternative means.

The amplified products are preferably identified as methylated or non-methylated with a probe or reporter specific to the product as described in US Patent 4,683,195 to Mullis et. al.*,* incorporated herein by reference in its entirety. Advances in the field of probes and reporters for detecting polynucleotides are well known to those skilled in the art. Optionally, the methylation pattern of the nucleic acid can be confirmed by other techniques such as restriction enzyme digestion and Southern blot analysis. Examples of methylation sensitive restriction endonucleases that can be used to detect 5'CpG methylation include SmaI, SacII, EagI, MspI, HpaII, BstUI and BssHII.

In another aspect of the invention a methylation ratio is used. This can be done by establishing a ratio between the amount of amplified methylated species of Marker attained and the amount of amplified reference marker or non-methylated Marker region amplified. PCR is most informative when it is quantitative real-time PCR. Ratios above an established or predetermined cutoff or threshold are considered hypermethylated and indicative of a poorer prognosis for recurrence than if the ratio does not exceed the cutoff. Cutoffs are established according to known methods in which such methods are used for at least two sets of samples: those with known condition of interest (recurrence or aggressiveness) and those with known normal conditions. The reference markers of the invention can also be used as internal controls. The reference marker is preferably a gene that is constitutively expressed in the cells of the samples such as Beta Actin.

Established or predetermined values (cutoff or threshold values) are also established and used in methods according to the invention in which a ratio is not used. In this case, the cutoff value is established with respect to the amount or degree of methylation relative to some baseline value such as the amount or degree of methylation in normal samples or in samples in which the cancer is clinically insignificant (is known not to progress to clinically relevant states or is not aggressive). These cutoffs are established according to well-known methods as in the case of their use in methods based on a methylation ratio.

The inventive methods and kits can include steps and reagents for multiplexing. That is, more than one Marker can be assayed at a time.

Interestingly, GSTP1, a Marker that is perhaps the most useful Marker for tumor assessment, was not found useful as surgical margin marker for the purposes of this invention. That is, it is not found in tumor margin samples even in patients with an aggressive or metastasized cancer. GSTP1 primers and probes that are referred to in the examples are:
**SEQ ID NO. 19 (GSTP1 SCORPION):**
   CGCACGGCGAACTCCCGCCGACGTGCG BHQ-HEG-TGTAGCGGTCGTCGGGGTTG
**SEQ ID NO. 20 (GSTPI SCORPION Antisense Primer):**
   GCCCCAATACTAAATCACGACG

Exemplary Markers found to be useful in the methods and kits of this invention are APC, RARβ2, and RASSF1A. Probes useful for detecting methylation of these areas are useful in such diagnostic or prognostic methods. Preferred molecules for the detection of Markers are shown below. The short name for the Marker gene is shown in parentheses along with the type of detection system employed. Antisense only refers to the orientation of the primer that is so designated in relationship to the priming sequence of the other member of the pair with which it is associated. It is not necessarily antisense with respect to genomic DNA.
**SEQ ID NO. 30 (RASSF1A SCORPION):**
   GCCGCGGTTTCGTTCGGTTCGCGGC-BHQ-HEG-CCCGTACTTCGCTAACTTTAAACG
**SEQ ID NO. 31 (RASSF1A SCORPION Antisense Primer):**
   GCGTTGAAGTCGGGGTTC
**SEQ ID NO. 32 (RARB2 SCORPION):**
   CGGCGCCCGACGATACCCAAAGCGCCG-BHQ-HEG-AACGCGAGCGATTCGAGTAG
**SEQ ID NO. 33 (RARB2 SCORPION Antisense Primer):**
   CTTACAAAAAACCTTCCGAATACG
**SEQ ID NO. 34 (APC SCORPION):**
   GCCGGCGGGTTTTCGACGGGCCGGC-BHQ-HEG-CGAACCAAAACGCTCCCCA
**SEQ ID NO. 35 (APC SCORPION Antisense Primer):**
   GTCGGTTACGTGCGTTTATATTTAG
**SEQ ID NO. 36 (ACTIN SCORPION):**
   GCGCCCAACCGCACAAGGGCGC-BHQ-HEG-GGGTATATTTTCGAGGGGTACG
**SEQ ID NO. 37 (ACTIN SCORPION Antisense Primer):**
   CGACCCGCACTCCGCAAT
**SEQ ID NO. 38(ACTIN SCORPION):**
   CCGCGCATCACCACCCCACACGCGCGG-BHQ-HEG-GGAGTATATAGGTTGGGGAAGTTTG
**SEQ ID NO. 39 (ACTIN SCORPION Antisense Primer):**
   AACACACAATAACAAACACAAATTCAC
**SEQ ID NO. 40 (ACTIN SCORPION):**
   CCCGGCTAAACCCACCATCCAGCCGGG-BHQ-HEG-GGGAGGGTAGTTTAGTTGTGGTT ,
**SEQ ID NO. 41 (ACTIN SCORPION Antisense Primer):**
   CAAAACAAAAAAACTAAATCTACACAACC
**SEQ ID NO. 42 (ACTIN SCORPION):**
   CCGCGGAACATTCAACTCAACCGCGG-BHQ-HEG-GGAGGAGGAAGGTAGGTTTTT
**SEQ ID NO. 43 (ACTIN SCORPION Antisense Primer):**
   ACATACAACAATCAATAACATAAAACCAC
**SEQ ID NO. 54 (HIN-1 SCORPION):**
   CGGCGCCCGAACCTCGAAAGCGCCG-BHQ-HEG-GGTTTTAGCGTTTGTTAAGAG
**SEQ ID NO. 55 (HIN-1 SCORPION Antisense Primer):**
   AACTTCCTACTACGACCGAC

### BHQ=Black Hole Quencher (BioSearch Technologies, San Fransisco, CA) HEG= Hexaethylene glycol

In a preferred embodiment of the invention, the Markers and methods described above are used in conjunction with histology information. When the two are combined, the false negative rate for histology drops appreciably. That is, when histology and the methods and Markers of the invention indicate a patient has a poor prognosis or aggressive disease, the probably is very high that such is the case. When histology alone provides no indication that a patient is likely to experience recurrence, metastasis, or aggressive progression of the disease, a positive finding of hypermethylation of the Markers suggests that histology results are inaccurate.

The kits of the invention can be configured with a variety of components provided that they all contain at least one primer or probe or a detection molecule (e.g., Scorpion reporter). In one embodiment, the kit includes reagents for amplifying and detecting hypermethylated Marker segments. Optionally, the kit includes sample preparation reagents and /or articles (e.g., tubes) to extract nucleic acids from samples.

In a preferred kit, reagents necessary for one-tube MSP are included such as, a corresponding PCR primer set, a thermostable DNA polymerase, such as Taq polymerase, and a suitable detection reagent(s) such as hydrolysis probe or molecular beacon. In optionally preferred kits, detection reagents are Scorpion reporters or reagents. A single dye primer or a fluorescent dye specific to double-stranded DNA such as ethidium bromide can also be used. The primers are preferably in quantities that yield high concentrations. Additional materials in the kit may include: suitable reaction tubes or vials, a barrier composition, typically a wax bead, optionally including magnesium; necessary buffers and reagents such as dNTPs; control nucleic acid (s) and/or any additional buffers, compounds, co-factors, ionic constituents, proteins and enzymes, polymers, and the like that may be used in MSP reactions. Optionally, the kits include nucleic acid extraction reagents and materials.

In a most preferred kit of the invention, instructions are included that provide that the assay is conducted on surgical margin samples and indicates where such samples are best obtained relative to the tumor core. In another kit according to the invention, the instructions provide that the surgical margin samples on which the assay is conducted are those which appear histophatolgically normal. The instructions may also indicate that a positive methylation result should be followed up with a particular course of therapy such as aggressive or prolonged treatment.

The materials for use in the assay of the invention are ideally suited for the preparation of a kit. Such a kit may comprise a carrier means being compartmentalized to receive in close confinement one or more container means such as vials, tubes, and the like, each of the container means comprising one of the separate elements to be used in the method.

For example, one of the container means may comprise a hybridization probe that is or can be detectably labeled. A second container may comprise a cell lysis buffer. The kit may also have containers holding nucleotide(s) for amplification of the target nucleic acid sequence and/or a container comprising a reporter-means, such as a biotin-binding protein, such as avidin or streptavidin, bound to a reporter molecule, such as an enzymatic, fluorescent, or radionuclide label.

### EXAMPLES

The examples describe studies and analyses of 48 lesions using samples from 37 prostatectomies. Ex vivo biopsy samples were generated from these, each extending outward from the core and separated approximately one mm apart in a linear array. The first core biopsy sample was confirmed histologically positive for cancer. Subsequent biopsies, one to four mm from the first, were confirmed histologically negative. The methylation ratio for the Markers was measured to adduce the magnitude and spatial dependence of a field effect.

### Example 1 (Sample Collection)

*Ex-vivo* biopsy cores were taken from retrospectively collected paraffin blocks of radical prostatectomy tissue from patients who underwent surgery within the last 5 years. A single core biopsy was taken from the center of the cancer lesion and 3 to 4 ex-vivo cores from the normal appearing proximal prostate tissue at an increasing distance of 1 mm from the central cancer lesion. Each biopsy core, ~ 1.5 mm in diameter and 3-4 mm deep, was re-embedded horizontally in paraffin blocks. Sections (5 x10 µ) were cut for methylation analysis, and bracketing H&E slides were prepared for each core and evaluated histologically.

### Example 2: Sample Preparation, DNA Isolation and Bisulfite Modification.

For DNA extraction 50µ tissue (5 x 10µ section) from each core block was deparaffinized in xylene (10 min) followed by 2 washes (5mins each) with 100% ethanol. After ethanol washes the tubes were incubated (with open lids) at 50 - 55 C in an oven to evaporate any residual ethanol. Deparaffinized tissue was incubated O/N in 40 µl of extraction buffer (10mM Tris, pH 8.0; 150 mM NaCl, 2mM EDTA, 0.5 % SDS) and 10 ul proteinase K (Qiagen) at 56 C in a heat block with shaking at 500 rpm. The next morning samples were heat inactivated at 70 °C x 10mins and spun at 16000x g for 5 mins. Fifty µl of tissue lysate (or 500 ng of universally methylated /unmethylated genomic control DNA) was processed for sodium bisulfite modification of DNA using an EZ DNA methylation kit (Zymo Research) according to the manufacturers protocol with the following modification in the incubation time and temperature. The samples were incubated at 70 °C for 3 hrs in a heat block with shaking at I 1 00rpm. The final elution of bisulfite treated DNA was done in 25 µl elution buffer. Samples were stored at-80 C. The modified DNA was used as template for quantitative methylation specific PCR.

### Example 3: Methylation Specific PCR (MSP)

Scorpion probes and primers were designed to amplify methylated CpG islands in the promoter regions of the genes of interest. A methylation-independent region was selected for amplification of the house keeping gene β-actin. The scorpion probe folding and amplicon probing was modeled using the DNA *mfold* program. Scorpion probes and primers were obtained from Scandinavian Gene Synthesis AB. The primer and probe sequences are identified below. For multiplex PCR, Scorpion probes for individual genes were labeled at the 5' position with a unique fluorescent dye (FAM, Cy5, Cy3 and Texas Red).

Calibration controls for each gene were made by amplifying the promoter sequence of interest for each gene from bisulfite-converted cell line DNA (MCF-7, LNCaP, and HCT-116) using methylation-specific primers. The amplified PCR product after clean up was cloned into TOPO TA cloning kit (Invitrogen) using standard protocols and transformed into *E coli.* Plasmid DNA was isolated, linearized and stored at - 80 C at a stock dilution of 2x 10⁷ copies /5 µl..Universally methylated and unmethylated genomic DNA (CpG-M and CpG-U) from Chemicon were used as positive and negative controls, respectively, for all PCR reactions.

Fluorogenic multiplex quantitative MSP assays were carried out on a Smart Cycler II (Cepheid, Sunnyvale CA) that enabled the simultaneous detection of 4 targets (using 4 fluorescence channels) within a single sample by using uniquely labeled specific probes for each gene. A 4 gene (GSTP1, APC, RAR -actin) and a 2 gene (RASSF1A, Beta-actin) multiplex MSP was performed for each sample and control DNA in duplicate tubes. The PCR reaction mix consisted of 500nM each of primer and probe for each gene, 5 U antibody coupled Fast start Taq Polymerase (Roche), 1.25 mM each of dATP, dCTP, dGTP and dTTP in a buffer mix (67.0 mM Tris, pH 8.8, 6.7 mM MgC12, 16.6mM (NH4)2SO4, 10mM -
llows: 95 C for 1min, followed by 40 cycles of 95 C for 30 s and 59 C for 30 s, with a 5 min extension at 72 C.

To generate copy number standard curves for each gene, a calibrator cocktail was freshly prepared by mixing equal volumes of calibrator stocks (2x10⁷ copies/5ul) for each gene in a tube. Ten fold serial dilutions (2x10⁶ to 2x10²) of this cocktail were made in water and run in duplicate in a PCR reaction as described above. The Cts (cycle threshold values) for each of the 4 genes was converted into copy numbers of methylated genes in each sample. The data for each sample is represented as a methylation ratio ([methylated copies of gene/copies of β-actin] x 1000). Standard curves were generated separately for the 4 gene and 2 gene multiplexes. Normalized % methylation was calculated by dividing the methylation ratio at each distance by the methylation ratio at the central, malignant core.

The Markers used in these multiplexes correspond to the following genes:
Multiplex 1: GSTP1 (Seq. ID. No 59), B-actin (Seq. ID. No. 60/61), APC (Seq. ID. No. 64/65), RARB2 (Seq. ID. No. 62)
Mutliplex 2: RASSF1A (Seq. ID. No. 69), B-actin (Seq. ID. No. 60/61)
Multiplex 3: Hin-1 (Seq. ID. No. 58), B-actin (Seq. ID. No. 60/61)
Primers and probes used in the assays were:
GSTP1: Probe :Seq. ID No. 19; Primer : Seq. ID No. 20
B-Actin: Probe :Seq. ID No. 38; Primer : Seq. ID No. 39
APC: Probe :Seq. ID No. 34; Primer : Seq. ID No. 35
RASSF1A: Probe :Seq. ID No. 30; Primer: Seq. ID No. 31
Hin-1: Probe :Seq. ID No. 54; Primer : Seq. ID No. 55

Table 1 below shows field effect observed in distant cores with each of the 5 genes. The data is shown as methylation ratio for each gene. Rigorous histopathology of the cores and surrounding areas was combined to qualify samples for field effect, the following criteria were used to qualify a lesion as fitting a 'field effect':
1. The cancer lesion (C0) has a positive methylation ratio for the gene.
2. All radial cores (C1 - C4) and the surrounding tissue have no histological evidence of cancer or high grade PIN.

**Table 1:**

| | | | Distance from Cancer lesion | | | |
|---|---|---|---|---|---|---|
| | Cases that fit model | Cases with Field Effect | 1 mm | 2mm | 3 mm | 4 mm |
| GSTP1 | 9 | 0 | 0 | 0 | 0 | 0 |
| APC | 8 | 1 | 0 | 1 | | |
| RARB2 | 11 | 4 | 3 | 1 | 2 | 0 |
| RASSF1A | 11 | 2 | 0 | 1 | 1 | 0 |
| HIN-1 | 11 | 5 | 1 | 2 | 1 | 1 |

From the data above, a field effect is seen for the Markers APC, RARB2, and RASSF1A and HIN-1. The field effect is seen at 2mm from the malignant core and in most cases up to 3 mm from the core. The strength of the signal generated by the detection of a methylated Marker generally varied as a function of the distance of the sample from the core but not in a linear fashion (Figures I and 2). This is likely due to the presence of multiple foci of cancerous events. Surprisingly, no field effect is seen for GSTP1.

## Claims

1. A method for characterizing prostate cancer in a patient comprising: obtaining a tissue sample external to a primary prostate neoplasm wherein said sample does not exhibit morphological characteristics of neoplastic pathology, determining the presence or quantity of a Marker in said sample, and assessing the cancer as aggressive if the amount or presence of said Marker exceeds a pre-determined value.

2. The method of claim 1 wherein the cancer is **characterized** as indolent if the amount or presence of said Marker does not exceed a pre-determined value.

3. The method of claim 1 wherein the Markers is selected from the group consisting of
-1.

4. A kit for conducting an assay for characterizing prostate cancer, comprising: Marker reagents.

5. The kit of claim 4 wherein the Marker is for a gene selected from the group consisting
-1.

6. The kit of claim 4 wherein the reagents include a member of the group consisting of Seq. ID No. 34 and 35.

7. The kit of claim 6 wherein the PCR priming reagents consist essentially of of Seq. ID No. 34 and 35.

8. The kit of claim 4 wherein the reagents include a member of the group consisting of Seq. ID No. 30 and 31.

9. The kit of claim 8 wherein the PCR priming reagents consist essentially of Seq. ID No. 30 and 31.

10. The kit of claim 4 wherein the reagents include a member of the group consisting of Seq. ID No. 32 and 33.

11. The kit of claim 10 wherein the PCR priming reagents consist essentially of Seq. ID No. 32 and 33.

12. The kit of claim 4 wherein the reagents include a member of the group consisting of Seq. ID No. 54 and 55.

13. The kit of claim 12 wherein the PCR priming reagents consist essentially of Seq. ID No. 54 and 55.

14. The kit of claim 4 further comprising reagents for amplifying and detecting the presence of a constitutively expressed gene.

15. The method of claim 1 further comprising establishing a methylation ratio and determining whether the methylation ratio exceeds a cutoff value.

16. The method of claim 1 for use in therapy monitoring.

17. The kit of claim 4 wherein the Markers consist essentially of Markers for RASSF1A and a constitutively expressed gene.

18. The kit of claim 17 wherein the constitutively expressed gene is B-Actin.

19. The kit of claim 4 wherein the Markers consist essentially of Marker for Hin-1 and a constitutively expressed gene.


SEQUENCE LISTING

<110>  MEHROTRA, Jyoti
       VARDE, Shobha
       MAZUMDER, Abhijit
       Gray, Karen
       Vargo, Janet

<120>  PROSTATE CANCER FIELD EFFECT ANALYSIS METHODS AND KITS

<130>  VDX5040USPSP

<140>  99/999999
<141>  2006-10-31

<160>  69

<170>  PatentIn version 3.3

<210>  1
<211>  46
<212>  DNA
<213>  Artificial

<220>
<223>  GSTP1 Scorpion


<220>
<221>  modified_base
<222>  (23)..(23)
<223>  -BHQ-HEG-

<220>
<221>  modified_base
<222>  (23)..(23)
<223>  -BHQ (Black Hole Quencher) -HEG (Hexaethylglycol) -

<400>  1
ccccgaacgt cgaccgctcg gggcgatttc ggggatttta gggcgt                    46


<210>  2
<211>  21
<212>  DNA
<213>  Artificial

<220>
<223>  GSTP1 Scorpion Antisense Primer

<400>  2
aaaatcccgc gaactcccgc c                                               21


<210>  3
<211>  46
<212>  DNA
<213>  Artificial

<220>
<223>  GSTP1 Scorpion


<220>
<221>  modified_base
<222>  (23)..(23)
<223>  -BHQ-HEG-

<400>  3
cccgaacgtc gaccgctttc gggcgatttc ggggatttta gggcgt                    46


<210>  4
<211>  21
<212>  DNA
<213>  Artificial

<220>
<223>  GSTP1 Scorpion Antisense Primer

<400>  4
aaaatcccgc gaactcccgc c                                               21


<210>  5
<211>  44
<212>  DNA
<213>  Artificial

<220>
<223>  GSTP1 Scorpion


<220>
<221>  modified_base
<222>  (22)..(22)
<223>  -BHQ-HEG-

<400>  5
cggcgggagt tcgcgggcgc cgactaaatc acgacgccga ccgc                      44


<210>  6
<211>  22
<212>  DNA
<213>  Artificial

<220>
<223>  GSTP1 Scorpion Antisense Primer

<400>  6
cggttagttg cgcggcgatt tc                                              22


<210>  7
<211>  41
<212>  DNA
<213>  Artificial

<220>
<223>  GSTP1 Scorpion


<220>
<221>  modified_base
<222>  (19)..(19)
<223>  -BHQ-HEG-

<220>
<221>  modified_base
<222>  (25)..(25)
<223>  -BHQ-HEG-

<400>  7
cgggagttcg cgggtcccga ctaaatcacg acgccgaccg c                         41

<210>  8
<211>  22
<212>  DNA
<213>  Artificial

<220>
<223>  GSTP1 Scorpion Antisense Primer

<400>  8
cggttagttg cgcggcgatt tc                                                22


<210>  9
<211>  50
<212>  DNA
<213>  Artificial

<220>
<223>  GSTP1 Scorpion


<220>
<221>  modified_base
<222>  (27)..(27)
<223>  -BHQ-HEG-

<400>  9
gtggttgatg tttggggtat caaccacaat cccacaaact cccaccaacc                  50


<210>  10
<211>  27
<212>  DNA
<213>  Artificial

<220>
<223>  GSTP1 Scorpion Antisense Primer

<400>  10
gtggtgattt tggggatttt agggtgt                                           27


<210>  11
<211>  47
<212>  DNA
<213>  Artificial

<220>
<223>  GSTP1 Scorpion


<220>
<221>  modified_base
<222>  (24)..(24)
<223>  -BHQ-HEG-

<400>  11
accccagtgg ttgatgtttg gggtaatccc acaaactccc accaacc                     47


<210>  12
<211>  27
<212>  DNA
<213>  Artificial

<220>
<223>  GSTP1 Scorpion

<400>  12
gtggtgattt tggggatttt agggtgt                                          27


<210>  13
<211>  56
<212>  DNA
<213>  Artificial

<220>
<223>  GSTP1 Scorpion


<220>
<221>  modified_base
<222>  (28)..(28)
<223>  -BHQ-HEG-

<400>  13
ccccacaggt tggtgggagt ttgtggggcc caatactaaa tcacaacacc aaccac          56


<210>  14
<211>  27
<212>  DNA
<213>  Artificial

<220>
<223>  GSTP1 Scorpion Antisense Primer

<400>  14
tggttagttg tgtggtgatt ttgggga                                          27


<210>  15
<211>  46
<212>  DNA
<213>  Artificial

<220>
<223>  GSTP1 Scorpion


<220>
<221>  modified_base
<222>  (23)..(23)
<223>  -BHQ-HEG-

<400>  15
ccccgaacgt cgaccgctcg gggcgatttc ggggatttta gggcgt                     46


<210>  16
<211>  21
<212>  DNA
<213>  Artificial

<220>
<223>  GSTP1 Scorpion Antisense Primer

<400>  16
aaaatcccgc gaactcccgc c                                                21


<210>  17
<211>  53
<212>  DNA

<213>  Artificial

<220>
<223>  GSTP1 Scorpion


<220>
<221>  modified_base
<222>  (30)..(30)
<223>  -BHQ-HEG-

<400>  17
cgcacgccga acgtcgaccg caaacgtgcg cgatttcggg gattttaggg cgt          53


<210>  18
<211>  21
<212>  DNA
<213>  Artificial

<220>
<223>  GSTP1 Scorpion Antisense Primer

<400>  18
aaaatcccgc gaactcccgc c                                             21


<210>  19
<211>  47
<212>  DNA
<213>  Artificial

<220>
<223>  GSTP1 Scorpion


<220>
<221>  modified_base
<222>  (27)..(27)
<223>  -BHQ-HEG-

<400>  19
cgcacggcga actcccgccg acgtgcgtgt agcggtcgtc ggggttg                 47


<210>  20
<211>  22
<212>  DNA
<213>  Artificial

<220>
<223>  GSTP1 Scorpion Antisense Primer

<400>  20
gccccaatac taaatcacga cg                                            22


<210>  21
<211>  55
<212>  DNA
<213>  Artificial

<220>
<223>  GSTP1 Scorpion


<220>
<221>  modified_base

<222>  (35)..(35)
<223>  -BHQ-HEG-

<400>  21
ccgacgcaca aaaaaacacc ctaaaatccg tcggggttag ttgtgtggtg atttt          55


<210>  22
<211>  20
<212>  DNA
<213>  Artificial

<220>
<223>  GSTP1 Scorpion Antisense Primer

<400>  22
cacaacacca accactcttc                                                  20


<210>  23
<211>  29
<212>  DNA
<213>  Artificial

<220>
<223>  GSTP1 Taqman Primer

<400>  23
cgtgatttag tattggggcg gagcggggc                                        29


<210>  24
<211>  25
<212>  DNA
<213>  Artificial

<220>
<223>  GSTP1 Taqman Primer

<400>  24
atccccgaaa aacgaaccgc gcgta                                            25


<210>  25
<211>  26
<212>  DNA
<213>  Artificial

<220>
<223>  GSTP1 Taqman Probe

<400>  25
tcggaggtcg cgaggttttc gttgga                                           26


<210>  26
<211>  48
<212>  DNA
<213>  Artifical


<220>
<221>  misc_feature
<223>  GSTP1 SCORPION

<220>
<221>  modified_base
<222>  (27)..(27)

<223>  -BHQ-HEG-

<400>  26
cggccctaaa accgctacga gggccggaag cgggtgtgta agtttcgg                48


<210>  27
<211>  26
<212>  DNA
<213>  Artifical


<220>
<221>  misc_feature
<223>  GSTP1 SCORPION Antisense Primer

<400>  27
acgaaatata cgcaacgaac taacgc                                        26


<210>  28
<211>  44
<212>  DNA
<213>  Artificial

<220>
<223>  GSTP1 SCORPION


<220>
<221>  misc_feature
<223>  GSTPI SCORPION

<220>
<221>  modified_base
<222>  (24)..(24)
<223>  -BHQ-HEG-

<400>  28
ccggtcgcga ggttttcgac cggccgaaaa acgaaccgcg cgta                    44


<210>  29
<211>  27
<212>  DNA
<213>  Artificial

<220>
<223>  GSTP1 SCORPION


<220>
<221>  misc_feature
<223>  GSTP1 SCORPION Antisense Primer

<400>  29
gggcgggatt atttttataa ggttcgg                                       27


<210>  30
<211>  49
<212>  DNA
<213>  Artificial

<220>
<223>  RASSFIA Scorpion

<220>
<221>  modified_base
<222>  (25)..(25)
<223>  -BHQ-HEG-

<400>  30
gccgcggttt cgttcggttc gcggccccgt acttcgctaa ctttaaacg                49


<210>  31
<211>  18
<212>  DNA
<213>  Artificial

<220>
<223>  RASSFIA Scorpion Antisense Primer

<400>  31
gcgttgaagt cggggttc                                                  18


<210>  32
<211>  47
<212>  DNA
<213>  Artifical


<220>
<221>  misc_feature
<223>  RARB2 SCORPION

<220>
<221>  modified_base
<222>  (27)..(27)
<223>  -BHQ-HEG-

<400>  32
cggcgcccga cgatacccaa agcgccgaac gcgagcgatt cgagtag                  47


<210>  33
<211>  24
<212>  DNA
<213>  Artificial

<220>
<223>  RARB2 SCORPION


<220>
<221>  misc_feature
<223>  RARB2 SCORPION Antisense Primer

<400>  33
cttacaaaaa accttccgaa tacg                                           24


<210>  34
<211>  44
<212>  DNA
<213>  Artificial

<220>
<223>  APC Scorpion


<220>
<221>  modified_base

<222>  (25)..(25)
<223>  -BHQ-HEG-

<400>  34
gccggcgggt tttcgacggg ccggccgaac caaaacgctc ccca                44


<210>  35
<211>  25
<212>  DNA
<213>  Artificial

<220>
<223>  APC Scorpion Antisense Primer

<400>  35
gtcggttacg tgcgtttata tttag                                     25


<210>  36
<211>  44
<212>  DNA
<213>  Artificial

<220>
<223>  Actin Scorpion


<220>
<221>  modified_base
<222>  (22)..(22)
<223>  -BHQ-HEG-

<400>  36
gcgcccaacc gcacaagggc gcgggtatat tttcgagggg tacg                44


<210>  37
<211>  18
<212>  DNA
<213>  Artificial

<220>
<223>  ACTIN Scorpion Antisense Primer

<400>  37
cgacccgcac tccgcaat                                             18


<210>  38
<211>  52
<212>  DNA
<213>  Artificial

<220>
<223>  ACTIN Scorpion


<220>
<221>  modified_base
<222>  (27)..(27)
<223>  -BHQ-HEG-

<400>  38
ccgcgcatca ccaccccaca cgcgcgggga gtatataggt tggggaagtt tg       52


<210>  39

<211>  27
<212>  DNA
<213>  Artificial

<220>
<223>  ACTIN Scorpion Antisense Primer

<400>  39
aacacacaat aacaaacaca aattcac                                          27


<210>  40
<211>  50
<212>  DNA
<213>  Artificial

<220>
<223>  ACTIN Scorpion


<220>
<221>  modified_base
<222>  (27)..(27)
<223>  -BHQ-HEG-

<400>  40
cccggctaaa cccaccatcc agccgggggg agggtagttt agttgtggtt                 50


<210>  41
<211>  29
<212>  DNA
<213>  Artificial

<220>
<223>  ACTIN Scorpion Antisense Primer

<400>  41
caaaacaaaa aaactaaatc tacacaacc                                        29


<210>  42
<211>  47
<212>  DNA
<213>  Artificial

<220>
<223>  ACTIN Scorpion


<220>
<221>  modified_base
<222>  (26)..(26)
<223>  -BHQ-HEG-

<400>  42
ccgcggaaca ttcaactcaa ccgcggggag gaggaaggta ggttttt                    47


<210>  43
<211>  29
<212>  DNA
<213>  Artificial

<220>
<223>  ACTIN Scorpion Antisense Primer

<400>  43

acatacaaca atcaataaca taaaaccac                                    29

<210>  44
<211>  47
<212>  DNA
<213>  Artificial

<220>
<223>  PTGS2/COX2 Scorpion

<220>
<221>  modified_base
<222>  (22)..(22)
<223>  -BHQ-HEG-

<400>  44
cacgccgccg tatctaggcg tggtttgttt cgacgtgatt ttttcga                47

<210>  45
<211>  21
<212>  DNA
<213>  Artificial

<220>
<223>  PTGS2/COX2 Antisense Primer

<400>  45
gcaaaaaatc ccctctcccg c                                            21

<210>  46
<211>  45
<212>  DNA
<213>  Artificial

<220>
<223>  PTGS2/COX2 Scorpion

<220>
<221>  modified_base
<222>  (22)..(22)
<223>  -BHQ-HEG-

<400>  46
gccgcgcaca aatttccgcg gcgaattggt tttcggaagc gttcg                  45

<210>  47
<211>  16
<212>  DNA
<213>  Artificial

<220>
<223>  PTGS2/COX2 Antisense Primer

<400>  47
cccgaattcc accgcc                                                  16

<210>  48
<211>  46
<212>  DNA
<213>  Artificial

<220>
<223>  PTGS2/COX2 Scorpion


<220>
<221>  modified_base
<222>  (23)..(23)
<223>  -BHQ-HEG-

<400>  48
ggcggaacgc acaaatttcc gccgaattgg ttttcggaag cgttcg                    46


<210>  49
<211>  16
<212>  DNA
<213>  Artificial

<220>
<223>  PTGS2/COX2 Antisense Primer

<400>  49
cccgaattcc accgcc                                                     16


<210>  50
<211>  51
<212>  DNA
<213>  Artificial

<220>
<223>  PTGS2/COX2 Scorpion


<220>
<221>  modified_base
<222>  (30)..(30)
<223>  -BHQ-HEG-

<400>  50
tgccgccgcc gtatctaatg gcggcagttt gtttcgacgt gattttttcg a               51


<210>  51
<211>  21
<212>  DNA
<213>  Artificial

<220>
<223>  PTGS2/COX2 Antisense Primer


<220>
<221>  misc_feature
<223>  PTGS2/COX2 Antisense Primer

<400>  51
gcaaaaaatc ccctctcccg c                                               21


<210>  52
<211>  39
<212>  DNA
<213>  Artificial

<220>
<223>  CDHI SCORPION

<220>
<221>  modified_base
<222>  (19)..(19)
<223>  -BHQ-HEG-

<400>  52
cgccgaatac gatcggcggt tcgttttagt tcggttcga                              39


<210>  53
<211>  18
<212>  DNA
<213>  Artificial

<220>
<223>  CDH1 SCORPION Antisense Primer

<400>  53
accgaaaacg ccgaacga                                                     18


<210>  54
<211>  46
<212>  DNA
<213>  Artificial

<220>
<223>  HIN-1 SCORPION


<220>
<221>  modified_base
<222>  (26)..(26)
<223>  -BHQ-HEG-

<400>  54
cggcgcccga acctcgaaag cgccgggttt tagcgtttgt taagag                      46


<210>  55
<211>  20
<212>  DNA
<213>  Artificial

<220>
<223>  HIN-1 SCORPIAN Antisense Primer

<400>  55
aacttcctac tacgaccgac                                                   20


<210>  56
<211>  2671
<212>  DNA
<213>  Homo sapiens


<220>
<221>  misc_feature
<223>  15LO1       >gi|187190|gb|M23892.1|HUMLOX15A Human 15-lipoxygenase mRNA,
        complete cds

<400>  56
aagatgggtc tctaccgcat ccgcgtgtcc actggggcct cgctctatgc cggttccaac      60

aaccaggtgc agctgtggct ggtcggccag cacggggagg cggcgctcgg gaagcgactg     120

tggcccgcac ggggcaagga gacagaactc aaggtggaag taccggagta tctggggccg 180
ctgctgtttg tgaaactgcg caaacggcac ctccttaagg acgacgcctg gttctgcaac 240
tggatctctg tgcagggccc cggagccggg gacgaggtca ggttcccttg ttaccgctgg 300
gtggagggca acggcgtcct gagcctgcct gaaggcaccg gccgcactgt gggcgaggac 360
cctcagggcc tgttccagaa acaccgggaa gaagagctgg aagagagaag gaagttgtac 420
cggtggggaa actggaagga cgggttaatt ctgaatatgg ctggggccaa actatatgac 480
ctccctgtgg atgagcgatt tctggaagac aagagagttg actttgaggt ttcgctggcc 540
aaggggctgg ccgacctcgc tatcaaagac tctctaaatg ttctgacttg ctggaaggat 600
ctagatgact tcaaccggat tttctggtgt ggtcagagca agctggctga gcgcgtgcgg 660
gactcctgga aggaagatgc cttatttggg taccagtttc ttaatggcgc caaccccgtg 720
gtgctgaggc gctctgctca ccttcctgct cgcctagtgt tccctccagg catggaggaa 780
ctgcaggccc agctggagaa ggagctggag ggaggcacac tgttcgaagc tgacttctcc 840
ctgctggatg ggatcaaggc caacgtcatt ctctgtagcc agcagcacct ggctgcccct 900
ctagtcatgc tgaaattgca gcctgatggg aaactcttgc ccatggtcat ccagctccag 960
ctgccccgca caggatcccc accacctccc cttttcttgc ctacggatcc cccaatggcc 1020
tggcttctgg ccaaatgctg ggtgcgcagc tctgacttcc agctccatga gctgcagtct 1080
catcttctga ggggacactt gatggctgag gtcattgttg tggccaccat gaggtgcctg 1140
ccgtcgatac atcctatctt caagcttata attccccacc tgcgatacac cctggaaatt 1200
aacgtccggg ccaggactgg gctggtctct gacatgggaa ttttcgacca gataatgagc 1260
actggtgggg gaggccacgt gcagctgctc aagcaagctg gagccttcct aacctacagc 1320
tccttctgtc cccctgatga cttggccgac cgggggctcc tgggagtgaa gtcttccttc 1380
tatgcccaag atgcgctgcg gctctgggaa atcatctatc ggtatgtgga aggaatcgtg 1440
agtctccact ataagacaga cgtggctgtg aaagacgacc cagagctgca gacctggtgt 1500
cgagagatca ctgaaatcgg gctgcaaggg gcccaggacc gagggtttcc tgtctcttta 1560
caggctcggg accaggtttg ccactttgtc accatgtgta tcttcacctg caccggccaa 1620
cacgcctctg tgcacctggg ccagctggac tggtactctt gggtgcctaa tgcaccctgc 1680
acgatgcggc tgcccccgcc aaccaccaag gatgcaacgc tggagacagt gatggcgaca 1740
ctgcccaact tccaccaggc ttctctccag atgtccatca cttggcagct gggcagacgc 1800
cagcccgtta tggtggctgt gggccagcat gaggaggagt atttttcggg ccctgagcct 1860
aaggctgtgc tgaagaagtt cagggaggag ctggctgccc tggataagga aattgagatc 1920
cggaatgcaa agctggacat gccctacgag tacctgcggc ccagcgtggt ggaaaacagt 1980
gtggccatct aagcgtcgcc accctttggt tatttcagcc cccatcaccc aagccacaag 2040
ctgacccctt cgtggttata gccctgccct cccaagtccc accctcttcc catgtcccac 2100
cctccctaga ggggcacctt ttcatggtct ctgcacccag tgaacacatt ttactctaga 2160

ggcatcacct gggaccttac tcctctttcc ttccttcctc ctttcctatc ttccttcctc 2220

tctctcttcc tctttcttca ttcagatcta tatggcaaat agccacaatt atataaatca 2280

tttcaagact agaatagggg gatataatac atattactcc acacctttta tgaatcaaat 2340

atgatttttt tgttgttgtt aagacagagt ctcactttga cacccaggct ggagtgcagt 2400

ggtgccatca ccacggctca ctgcagcctc agcgtcctgg gctcaaatga tcctcccacc 2460

tcagcctcct gagtagctgg gactacaggc tcatgccatc atgcccagct aatatttttt 2520

tattttcgtg gagacggggc ctcactatgt tgcctaggct ggaaatagga ttttgaaccc 2580

aaattgagtt taacaataat aaaaagttgt tttacgctaa agatggaaaa gaactaggac 2640

tgaactattt taaataaaat attggcaaaa g 2671

<210> 57
<211> 1193
<212> DNA
<213> Homo sapiens

<220>
<221> misc_feature
<223> CDH1 >gi|509604|gb|L34545.1|HUMECADN Human E-cadherin gene, promoter
region and 5' end

<400> 57
   tctagaaaaa ttttttaaaa aattaggccg ctcgagcgag agtgcagtgg ctcacgcctg 60
   taatccaaca cttcaggagg ctgaagaggg tggatcacct gaggtcagga gttccagacc 120
   agcctggcca acatggtgaa accccgtctt gtactaaaaa tacaaaatta gccggtgtgg 180
   tggcacacgc ctgtagtccc agctactcaa taggctgaga caggagagtc tcttgaaccc 240
   ggcaggcgga ggttgcagtg agccgagatc gtgccactgc actccagcct gggcaagaca 300
   gagcgagact ccgtctcaaa aaatacaaac aaaacaaaca aacaaaaaat taggctgcta 360
   gctcagtggc tcatggctca cacctgaaat cctagcactt tgggaggcca aggcaggagg 420
   atcgcttcag cccaggagtt cgagaccagg ctgggcaata cagggagaca cagcgccccc 480
   actgcccctg tccgccccga cttgtctctc tacaaaaagg caaaagaaaa aaaaaattag 540
   cctggcgtgg tggtgtgcac ctgtactccc agctactaga gaggctgggg ccagaggacc 600
   gcttgagccc aggagttcga ggctgcagtg agctgtgatc gcaccactgc actccagctt 660
   gggtgaaaga gtgagcccca tctccaaaac gaacaaacaa aaaatcccaa aaaacaaaag 720
   aactcagcca agtgtaaaag ccctttctga tcccaggtct tagtgagcca ccggcggggc 780
   tgggattcga acccagtgga atcagaaccg tgcaggtccc ataacccacc tagaccctag 840
   caactccagg ctagagggtc accgcgtcta tgcgaggccg ggtgggcggg ccgtcagctc 900
   cgccctgggg aggggtccgc gctgctgatt ggctgtggcc ggcaggtgaa ccctcagcca 960
   atcagcggta cggggggcgg tgctccgggg ctcacctggc tgcagccacg caccccctct 1020
   cagtggcgtc ggaactgcaa agcacctgtg agcttgcgga agtcagttca gactccagcc 1080
   cgctccagcc cggcccgacc cgaccgcacc cggcgcctgc cctcgctcgg cgtccccggc 1140
   cagccatggg cccttggagc cgcagcctct cggcgctgct gctgctgctg cag 1193

<210> 58
<211> 2635
<212> DNA
<213> Homo sapiens

<220>
<221> misc_feature
<223> Hin-1 REGION ON CHROMOSOME 5( Alternate name: (SCGB3A1)

<220>
<221> misc_feature
<223> This gene can be found on Chromosome 5 at location
179,949,712-179,951,146. The start of this gene is located in
Contig AC122714.2.1.190024.
>chromosome:NCBI35:5:179949112:179951746:-1

<400> 58
atctgtcata cttctgcagg tgacagacga gtgaggacat ttagagaaac tcaggaacaa 60

accaggccca tccactggcg tcgaggctag tttcgaagac agaaaaacgc cccacttctg 120

tttgcactgt ggctgcctgt ggcccggccg gggaggcggc cgggagtgag gcctgatcgt 180

ccctggcgcc tccacctccc caggcgcaga aggcgcccac gaggaccccc agtgcccgac 240

gttgccacgg tctgggatca gaggcaggga ccagggagcc aggaactgcg ccgcccccgc 300

ccctgccctg gcgcgaggga agctcccctc acccgggccc agccctgcag gggggcgcgt 360

ggggtcagac cgcaaagcga aggtgcgggc cggggtgggc ctcgcggaga caaaggccgg 420

gcctgcctgc tctcagaggg ccccagcgcc tgccaagagg aagtcctcga ggcccgggca 480

gggaaggggg cacgggcttc ccagggcccg ccggccgcag caggaagttg gccagggcac 540

ggccgtgagc ggagcgggca gggctttctc aggagcgcgg gcgaggccgg cgctggaggg 600

gcgaggaccg ggtataagaa gcctcgtggc cttgcccggg cagccgcagg ttccccgcgc 660

gccccgagcc cccgcgccat gaagctcgcc gccctcctgg ggctctgcgt ggccctgtcc 720

tgcagctccg gtgagcgccc cgggctcctg gcgcgcacgg tggggcctga ggcctcggcg 780

cccgctgcgc ccccgccgct gcctgcgccg atcgtggatc ccaggtcctt ccagcctcgc 840

ccggcgccca gagggctccg ccaccccggg gccccgcgcc tgcagcccgc ggcctccccc 900

tctcagggct gtctccagcc tcgtgccggg atggaggccg cccctggcct ggggacaccc 960

gtctgccccc cgtctggaga ccggctcctc atcctgcaag gcgcagcgcg agtgtcccct 1020

cccttggggg cctgtcccgg accctgcaca gagttcaccg gtccttccgc caccctcagg 1080

ccactccggt gaccctgcag cgtctcctgg cggggccgcc tccccagacc cgcctgtgtc 1140

ccgggggctc gcacctggca ggcctcggcg caggagggag gggcggtcgg ggagccgggc 1200

agggcgcgac ggttcctggg cgcctcccgc gggggcgcgt cctggacctg ccttctgggg 1260

accccggcgc gcaggcggtg acccctccct gttcgcttgc agctgctgct ttcttagtgg 1320

gctcggccaa gcctgtggcc cagcctgtcg ctgcgctgga gtcggcggcg gaggccgggg 1380

ccgggaccct ggccaacccc ctcggcaccc tcaacccgct gaagctcctg ctgagcagcc 1440

tgggcatccc cgtgaaccac ctcatagagg gctcccagaa gtgtgtggct gagctgggtc 1500

cccaggccgt gggggccgtg aaggccctga aggccctgct ggtaaagtgg gcaccccggg 1560

tgcccttcct gcgcgggcat cccttcccgg ccagcctcaa aactgcacca gaggtgtccc 1620

ggccctacgt cagggctgtt tcccgtcctg cccctccaag ccctgtccct gggagaagcc 1680

cgggtctccg ggtgggaact gggtggggcg catccgagct gcaggggcgg ggaaggcgga 1740

gatggccccg cgcgggtgcg cgtcccccgg agacgccagc ggaaccgccc cgctcccgct 1800

ccccacagag ccggccgctg ccgcgccccc gcctgagttc ttggtggcag ggctgggggc 1860

actcaagctc ggcttctgct ttccaggggg ccctgacagt gtttggctga gccgagactg 1920

gagcatctac acctgaggac aagacgctgc ccacccgcga gggctgaaaa ccccgccgcg 1980

gggaggaccg tccatcccct tcccccggcc cctctcaata aacgtggtta agagcagctc 2040

gagcctggta ttttctcatc caaatgctga tccctgtggg ggaccgagag tgccaagtcc 2100

cagattttgt gaggggtgct gcgtgagaga gggagagaga ggccgcgaga gtctgcaggt 2160

ggcatagagc cagcagtgca aggtgacaga gatggggcca gtatcagagg taaaccggat 2220

ttggaaacaa aactggcagg tctttcttcc tgttgtgctc tgtaactgtt tatgaagcat 2280

aaaaattatc tactccttga agactggcga gaatgcaaac gcaccaaagc catttgttgc 2340

taaaatttga aagtttttat tttttccctc tatacttgta gatttcttcc tcggaatggg 2400

ggaggaatga gggcttcagg aaatccgctg ttgggcttgt tttccctgca gtttctgctg 2460

gttttgtcca aataacctag tgactttgat tccttctact ctagcggaga gaccaccttt 2520

tgtcaactga tctagtgttg gtgggaaggt gcccagggaa cccagacatc ggtgtccctt 2580

tcaggggaag cctcccagtt gacttccttg ctgggatgaa taactctgcc tcagg 2635

<210> 59
<211> 4260
<212> DNA
<213> Homo sapiens

<220>
<221> misc_feature
<223> >gi|341173|gb|M24485.1|HUMGSTP1G Homo sapiens (clone pHGST-pi)
glutathione S-transferase pi (GSTP1) gene, complete cds

<400> 59
   aacaagagat caatatctag aataaatgga gatctgcaaa tcaacagaaa gtaggcagca 60
   aagccaaaga aaatagccta aggcacagcc actaaaagga acgtgatcat gtcctttgca 120
   gggacatggg tggagctgga agccgttagc ctcagcaaac tcacacagga acagaaaacc 180
   agcgagaccg catggtctca cttataagtg ggagctgaac aatgagaaca catggtcaca 240
   tggcggcgat caacacacac tggtgcctgt tgagcggggt gctggggagg gagagtacca 300
   ggaagaatag ctaagggata ctgggcttaa tacctgggtg atgggatgat ctgtacagca 360
   aaccatcatg gcgcacacac ctatgtaaca aacctgcaca tcctgcacat gtaccccaga 420
   acttcaaata aaagttggac ggccaggcgt ggtggctcac gcctgtaatc ccagcacttt 480
   gggaagccga ggcgtgcaga tcacctaagg tcaggagttc gagaccagcc cggccaacat 540
   ggtgaaaccc cgtctctact aaaaatacaa aaatcagcca gatgtggcac gcacctataa 600
   ttccacctac tcgggaggct gaagcagaat tgcttgaacc cgagaggcgg aggttgcagt 660
   gagccgccga gatcgcgcca ctgcactcca gcctgggcca cagcgtgaga ctacgtcata 720
   aaataaaata aaataacaca aaataaaata aaataaaata aaataaaata aaataataaa 780
   ataaaataaa ataaaataaa ataaaataaa ataaagcaat ttcctttcct ctaagcggcc 840
   tccacccctc tcccctgccc tgtgaagcgg gtgtgcaagc tccgggatcg cagcggtctt 900
   agggaatttc cccccgcgat gtcccggcgc gccagttcgc tgcgcacact tcgctgcggt 960
   cctcttcctg ctgtctgttt actccctagg ccccgctggg gacctgggaa agagggaaag 1020
   gcttccccgg ccagctgcgc ggcgactccg gggactccag ggcgcccctc tgcggccgac 1080
   gcccggggtg cagcggccgc cggggctggg gccggcggga gtccgcggga ccctccagaa 1140
   gagcggccgg cgccgtgact cagcactggg gcggagcggg gcgggaccac ccttataagg 1200
   ctcggaggcc gcgaggcctt cgctggagtt tcgccgccgc agtcttcgcc accagtgagt 1260
   acgcgcggcc cgctccccgg ggatggggct cagagctccc agcatggggc caacccgcag 1320
   catcaggccc gggctcccgg cagggctcct cgcccacctc gagacccggg acgggggcct 1380
   aggggaccca ggacgtcccc agtgccgtta gcggctttca gggggcccgg agcgcctcgg 1440
   ggagggatgg gaccccgggg gcggggaggg ggggcaggct gcgctcaccg cgccttggca 1500
   tcctcccccg ggctccagca aacttttctt tgttcgctgc agtgccgccc tacaccgtgg 1560
   tctatttccc agttcgaggt aggagcatgt gtctggcagg gaagggaggc aggggctggg 1620
   gctgcagccc acagcccctc gcccacccgg agagatccga acccccttat ccctccgtcg 1680
   tgtggctttt accccgggcc tccttcctgt tccccgcctc tcccgccatg cctgctcccc 1740
   gccccagtgt tgtgtgaaat cttcggagga acctgtttac ctgttccctc cctgcactcc 1800
   tgacccctcc ccgggttgct gcgaggcgga gtcggcccgg tccccacatc tcgtacttct 1860
   ccctccccgc aggccgctgc gcggccctgc gcatgctgct ggcagatcag ggccagagct 1920
   ggaaggagga ggtggtgacc gtggagacgt ggcaggaggg ctcactcaaa gcctcctgcg 1980
   taagtgacca tgcccgggca aggggagggg gtgctgggcc ttagggggct gtgactagga 2040
   tcgggggacg cccaagctca gtgcccctcc ctgagccatg cctcccccaa cagctatacg 2100
   ggcagctccc caagttccag gacggagacc tcaccctgta ccagtccaat accatcctgc 2160
   gtcacctggg ccgcaccctt ggtgagtctt gaacctccaa gtccagggca ggcatgggca 2220
   agcctctgcc cccggagccc ttttgtttaa atcagctgcc ccgcagccct ctggagtgga 2280
   ggaaactgag acccactgag gttacgtagt ttgcccaagg tcaagcctgg gtgcctgcaa 2340
   tccttgccct gtgccaggct gcctcccagg tgtcaggtga gctctgagca cctgctgtgt 2400
   ggcagtctct catccttcca cgcacatcct cttcccctcc tcccaggctg gggctcacag 2460
   acagccccct ggttggccca tccccagtga ctgtgtgttg atcaggcgcc cagtcacgcg 2520
   gcctgctccc ctccacccaa ccccagggct ctatgggaag gaccagcagg aggcagccct 2580
   ggtggacatg gtgaatgacg gcgtggagga cctccgctgc aaatacatct ccctcatcta 2640
   caccaactat gtgagcatct gcaccagggt tgggcactgg gggctgaaca aagaaagggg 2700
   cttcttgtgc cctcaccccc cttacccctc aggtggcttg ggctgacccc ttcttgggtc 2760
   agggtgcagg ggctgggtca gctctgggcc aggggcccag gggcctggga caagacacaa 2820
   cctgcaccct tattgcctgg gacatcaacc agccaagtaa cgggtcatgg gggcgagtgc 2880
   aaggacagag acctccagca actggtggtt tctgatctcc tggggtggcg agggcttcct 2940
   ggagtagcca gaggtggagg aggatttgtc gccagtttct ggatggaggt gctggcactt 3000
   ttagctgagg aaaatatgca gacacagagc acatttgggg acctgggacc agttcagcag 3060
   aggcagcgtg tgtgcgcgtg cgtgtgcgtg tgtgtgcgtg tgtgtgtgta cgcttgcatt 3120
   tgtgtcgggt gggtaaggag atagagatgg gcgggcagta ggcccaggtc ccgaaggcct 3180
   tgaacccact ggtttggagt ctcctaaggg caatgggggc cattgagaag tctgaacagg 3240
   gctgtgtctg aatgtgaggt ctagaaggat cctccagaga agccagctct aaagcttttg 3300
   caatcatctg gtgagagaac ccagcaagga tggacaggca gaatggaata gagatgagtt 3360
   ggcagctgaa gtggacagga tttggtacta gcctggttgt ggggagcaag cagaggagaa 3420
   tctgggactc tggtgtctgg cctggggcag acgggggtgt ctcaggggct gggagggatg 3480
   agagtaggat gatacatggt ggtgtctggc aggaggcggg caaggatgac tatgtgaagg 3540
   cactgcccgg gcaactgaag ccttttgaga ccctgctgtc ccagaaccag ggaggcaaga 3600
   ccttcattgt gggagaccag gtgagcatct ggccccatgc tgttccttcc tcgccaccct 3660
   ctgcttccag atggacacag gtgtgagcca tttgtttagc aaagcagagc agacctaggg 3720
   gatgggctta ggccctctgc ccccaattcc tccagcctgc tcccgctggc tgagtcccta 3780
   gcccccctgc cctgcagatc tccttcgctg actacaacct gctggacttg ctgctgatcc 3840
   atgaggtcct agcccctggc tgcctggatg cgttccccct gctctcagca tatgtggggc 3900
   gcctcagtgc ccggcccaag ctcaaggcct tcctggcctc ccctgagtac gtgaacctcc 3960
   ccatcaatgg caacgggaaa cagtgagggt tgggggactc ctgagcggga ggcagagttt 4020
   gccttccttt ctccaggacc aataaaattt ctaagagagc tactatgagc actgtgtttc 4080
   ctgggacggg gcttaggggt tctcagcctc gaggtcggtg ggagggcaga gcagaggact 4140
   agaaaacagc tcctccagca cagtcagtgg cttcctggag ccctcagcct ggctgtgttt 4200
   actgaacctc acaaactaga agaggaagaa aaaaaaagag agagagaaac aaagagaaat 4260

<210> 60
<211> 2011
<212> DNA
<213> Homo sapiens

<220>
<221>  misc_feature
<223>  B-Actin      >gi|28337|emb|Y00474.1|HSACTBPR Human beta-actin gene
5'-flanking
      region, CpG Island 1656 to 1955

<400>  60
   gagctctgtc tcttggccag ctgaatggag gcccagcggc aacacaggtc ctgcctgggg      60
   atcaggtctg ctctgcaccc caccttgctg cctggagccg cccacctgac aacctctcat     120
   ccctgctctg tagatccggt cccatcccca ctgcccaccc caccccccca gcactccacc     180
   cagttcaacg ttccacgaac ccccagaacc agccctcatc aacaggcagc aagaagggcc     240
   ccccgcccat cgccccacaa cgccagccgg gtgaactgta gcgttggcag gtcctgaggc     300
   agctgaaaga tacaaggcca gggacaggac agtcccatcc ccaggaggca gggagtatac     360
   aggctgggga agtttgccct tgcgtggggt ggtgatggag gaggctcagc aagtcttctg     420
   gactgtgaac ctgtgtctgc cactgtgtgc tgggtggtgg tcatctttcc caccaggctg     480
   tggcctctgc aaccttcaag ggaggagcag gtcccattgg ctgagcacag ccttgtacgt     540
   gaactgaaca agcagcctcc ttcctggcca caggttccat gtccttatat ggactcatct     600
   ttgcctattg cgacacacac tcaatgaaca cctactacgc gctgcaaaga gccccgcagg     660
   cctgaggtgc ccccacctca ccactcttcc tatttttgtg taaaaatcca gcttcttgtc     720
   accacctcca aggaggggga ggaggaggaa ggcaggttcc tctaggctga gccgaatgcc     780
   cctctgtggt cccacgccac tgatcgctgc atgcccacca cctgggtaca cacagtctgt     840
   gattcccgga gcagaacgga ccctgcccac ccggtcttgt gtgctactca gtggacagac     900
   ccaaggcaag aaagggtgac aaggacaggg tcttcccagg ctggctttga gttcctagca     960
   ccgccccgcc cccaatcctc tgtggcacat ggagtcttgg tccccagagt cccccagcgg    1020
   cctccagatg gtctgggagg gcagttcagc tgtggctgcg catagcagac atacaacgga    1080
   cggtgggccc agacccaggc tgtgtagacc cagccccccc gccccgcagt gcctaggtca    1140
   cccactaacg ccccaggcct ggtcttggct gggcgtgact gttaccctca aaagcaggca    1200
   gctccagggt aaaaggtgcc ctgccctgta gagcccactt ccttcccagg gctgcggctg    1260
   ggtaggtttg tagccttcat cacgggccac ctccagccac tggaccgctg gcccctgccc    1320
   tgtcctgggg agtgtggtcc tgcgactcta atggccgcaa gccacctgac tcccccaaca    1380
   ccacactcta cctctcaagc ccaggtctct ccctagtgac ccacccagca catttagcta    1440
   gctgagcccc acagccagag gtcctcaggc cctgctttca gggcagttgc tctgaagtcg    1500
   gcaaggggga gtgactgcct ggccactcca tgccctccaa gagctccttc tgcaggagcg    1560
   tacagaaccc agggccctgg cacccgtgca gaccctggcc caccccacct gggcgctcag    1620
   tgcccaagag atgtccacac ctaggatgtc ccgcggtggg tggggggccc gagagacggg    1680
   caggccgggg gcaggcctgg ccatgcgggg ccgaaccggg cactgcccag cgtggggcgc    1740
   gggggccacg gcgcgcgccc ccagcccccg ggcccagcac cccaaggcgg ccaacgccaa    1800
   aactctccct cctcctcttc ctcaatctcg ctctcgctct tttttttttt cgcaaaagga 1860
   ggggagaggg ggtaaaaaaa tgctgcactg tcggcgaagc cggtgagtga gcggcgcggg 1920
   gccaatcgcg tgcgccgttc cgaaagttgc cttttatggc tcgagcggcc gcggcggcgc 1980
   cctataaaac ccagcggcgc gacgcgccac c 2011

<210> 61
<211> 1792
<212> DNA
<213> Homo sapiens

<220>
<221> misc_feature
<223> >gi|5016088|ref|NM_001101.2| Homo sapiens actin, beta (ACTB),
      mRNA

<400> 61
cgcgtccgcc ccgcgagcac agagcctcgc ctttgccgat ccgccgcccg tccacacccg 60

ccgccagctc accatggatg atgatatcgc cgcgctcgtc gtcgacaacg gctccggcat 120

gtgcaaggcc ggcttcgcgg gcgacgatgc cccccgggcc gtcttcccct ccatcgtggg 180

gcgccccagg caccagggcg tgatggtggg catgggtcag aaggattcct atgtgggcga 240

cgaggcccag agcaagagag gcatcctcac cctgaagtac cccatcgagc acggcatcgt 300

caccaactgg gacgacatgg agaaaatctg gcaccacacc ttctacaatg agctgcgtgt 360

ggctcccgag gagcaccccg tgctgctgac cgaggccccc ctgaacccca aggccaaccg 420

cgagaagatg acccagatca tgtttgagac cttcaacacc ccagccatgt acgttgctat 480

ccaggctgtg ctatccctgt acgcctctgg ccgtaccact ggcatcgtga tggactccgg 540

tgacggggtc acccacactg tgcccatcta cgaggggtat gccctccccc atgccatcct 600

gcgtctggac ctggctggcc gggacctgac tgactacctc atgaagatcc tcaccgagcg 660

cggctacagc ttcaccacca cggccgagcg ggaaatcgtg cgtgacatta aggagaagct 720

gtgctacgtc gccctggact tcgagcaaga gatggccacg gctgcttcca gctcctccct 780

ggagaagagc tacgagctgc ctgacggcca ggtcatcacc attggcaatg agcggttccg 840

ctgccctgag gcactcttcc agccttcctt cctgggcatg gagtcctgtg gcatccacga 900

aactaccttc aactccatca tgaagtgtga cgtggacatc cgcaaagacc tgtacgccaa 960

cacagtgctg tctggcggca ccaccatgta ccctggcatt gccgacagga tgcagaagga 1020

gatcactgcc ctggcaccca gcacaatgaa gatcaagatc attgctcctc ctgagcgcaa 1080

gtactccgtg tggatcggcg gctccatcct ggcctcgctg tccaccttcc agcagatgtg 1140

gatcagcaag caggagtatg acgagtccgg cccctccatc gtccaccgca aatgcttcta 1200

ggcggactat gacttagttg cgttacaccc tttcttgaca aaacctaact tgcgcagaaa 1260

acaagatgag attggcatgg ctttatttgt ttttttttgt tttgttttgg tttttttttt 1320

tttttggctt gactcaggat ttaaaaactg gaacggtgaa ggtgacagca gtcggttgga 1380

gcgagcatcc cccaaagttc acaatgtggc cgaggacttt gattgcacat tgttgttttt 1440

ttaatagtca ttccaaatat gagatgcatt gttacaggaa gtcccttgcc atcctaaaag 1500

ccaccccact tctctctaag gagaatggcc cagtcctctc ccaagtccac acaggggagg 1560

tgatagcatt gctttcgtgt aaattatgta atgcaaaatt tttttaatct tcgccttaat 1620

actttttat tttgttttat tttgaatgat gagccttcgt gcccccctt cccccttttt 1680

gtcccccaac ttgagatgta tgaaggcttt tggtctccct gggagtgggt ggaggcagcc 1740

agggcttacc tgtacactga cttgagacca gttgaataaa agtgcacacc tt 1792

<210> 62
<211> 2762
<212> DNA
<213> Homo sapiens

<220>
<221> misc_feature
<223> RARB2 >gi|14916495|ref|NM_016152.2| Homo sapiens retinoic acid
 receptor, beta (RARB), transcript variant 2, mRNA

<400> 62
gtgacagaag tagtaggaag tgagctgttc agaggcagga gggtctattc tttgccaaag 60

gggggaccag aattccccat gcgagctgtt tgaggactgg gatgccgaga acgcgagcga 120

tccgagcagg gtttgtctgg gcaccgtcgg ggtaggatcc ggaacgcatt cggaaggctt 180

tttgcaagca tttacttgga aggagaactt gggatctttc tgggaacccc ccgccccggc 240

tggattggcc gagcaagcct ggaaaatgca attgaaacac agagcaccag ctctgaggaa 300

ctcgtcccaa gcccccatc tccacttcct ccccctcgag tgtacaaacc ctgcttcgtc 360

tgccaggaca aatcatcagg gtaccactat ggggtcagcg cctgtgaggg atgtaagggc 420

tttttccgca gaagtattca gaagaatatg atttacactt gtcaccgaga taagaactgt 480

gttattaata aagtcaccag gaatcgatgc caatactgtc gactccagaa gtgctttgaa 540

gtgggaatgt ccaaagaatc tgtcaggaat gacaggaaca agaaaaagaa ggagacttcg 600

aagcaagaat gcacagagag ctatgaaatg acagctgagt tggacgatct cacagagaag 660

atccgaaaag ctcaccagga aactttccct tcactctgcc agctgggtaa atacaccacg 720

aattccagtg ctgaccatcg agtccgactg gacctgggcc tctgggacaa attcagtgaa 780

ctggccacca agtgcattat taagatcgtg gagtttgcta aacgtctgcc tggtttcact 840

ggcttgacca tcgcagacca aattaccctg ctgaaggccg cctgcctgga catcctgatt 900

cttagaattt gcaccaggta taccccagaa caagacacca tgactttctc agacggcctt 960

accctaaatc gaactcagat gcacaatgct ggatttggtc ctctgactga ccttgtgttc 1020

acctttgcca accagctcct gcctttggaa atggatgaca cagaaacagg ccttctcagt 1080

gccatctgct taatctgtgg agaccgccag gaccttgagg aaccgacaaa agtagataag 1140

ctacaagaac cattgctgga agcactaaaa atttatatca gaaaaagacg acccagcaag 1200

cctcacatgt ttccaaagat cttaatgaaa atcacagatc tccgtagcat cagtgctaaa 1260

ggtgcagagc gtgtaattac cttgaaaatg gaaattcctg gatcaatgcc acctctcatt 1320
caagaaatgc tggagaattc tgaaggacat gaacccttga ccccaagttc aagtgggaac 1380
acagcagagc acagtcctag catctcaccc agctcagtgg aaaacagtgg ggtcagtcag 1440
tcaccactcg tgcaataaga cattttctag ctacttcaaa cattccccag taccttcagt 1500
tccaggattt aaaatgcaag aaaaaacatt tttactgctg cttagttttt ggactgaaaa 1560
gatattaaaa ctcaagaagg accaagaagt tttcatatgt atcaatatat atactcctca 1620
ctgtgtaact tacctagaaa tacaaacttt tccaatttta aaaaatcagc catttcatgc 1680
aaccagaaac tagttaaaag cttctatttt cctctttgaa cactcaagat gcatggcaaa 1740
gacccagtca aaatgattta cccctggtta agtttctgaa gactttgtac atacagaagt 1800
atggctctgt tctttctata ctgtatgttt ggtgctttcc ttttgtcttg catactcaaa 1860
ataaccatga caccaaggtt atgaaataga ctactgtaca cgtctaccta ggttcaaaaa 1920
gataactgtc ttgctttcat ggaatagtca agacatcaag gtaaggaaac aggactattg 1980
acaggactat tgtacagtat gacaagataa ggctgaagat attctacttt agttagtatg 2040
gaagcttgtc tttgctcttt ctgatgctct caaactgcat cttttatttc atgttgccca 2100
gtaaaagtat acaaattccc tgcactagca gaagagaatt ctgtatcagt gtaactgcca 2160
gttcagttaa tcaaatgtca tttgttcaat tgttaatgtc actttaaatt aaaagtggtt 2220
tattacttgt ttaatgacat aactacacag ttagttaaaa aaaatttttt tacagtaatg 2280
atagcctcca aggcagaaac acttttcagt gttaagtttt tgtttacttg ttcacaagcc 2340
attagggaaa tttcatggga taattagcag gctggtctac cactggacca tgtaactcta 2400
gtgtccttcc tgattcatgc ctgatattgg gatttttttc cagcccttct tgatgccaag 2460
ggctaattat attacatccc aaagaaacag gcatagaatc tgcctccttt gaccttgttc 2520
aatcactatg aagcagagtg aaagctgtgg tagagtggtt aacagataca agtgtcagtt 2580
tcttagttct catttaagca ctactggaat tttttttttt gatatattag caagtctgtg 2640
atgtactttc actggctctg tttgtacatt gagattgttt gtttaacaat gctttctatg 2700
ttcatatact gtttaccttt ttccatggac tctcctggca aagaataaaa tatatttatt 2760
tt 2762

<210> 63
<211> 5487
<212> DNA
<213> Homo sapiens

<220>
<221> misc_feature
<223> TIMP3 >gi|21536431|ref|NM_000362.3| Homo sapiens tissue inhibitor
of
metalloproteinase 3 (Sorsby fundus dystrophy, pseudoinflammatory)
(TIMP3), mRNA

<400> 63
tctgtcgact tgccccagag ctgatccttg tctttgtcca cttctcagcg aggatggcac 60

ttcagggagc ccttccctta ctatcgcaga gagagcaggc cctccccagt catgtccaac 120
ccagaactct gttttgtttt cttcatagcc ctagcatcac agaaaatcac cctgtgcatt 180
catggatgtc cacgggggca agggctttgt gttgcttaac ccagcatcct gaaccgtgtt 240
tgttgaatga atacagaacc ccgtttgctc tgggagagca cagaaaacag tcttctatca 300
tatatcatag ccagctgcaa acagcagatg gcttcccata tcccagagag taagaaccag 360
agagagagag aaagagagag agtttgggtc tttctcctct gtgcctgctc tctccagaga 420
aactggaggg gtagcagtta gcattccccc gctggttcca ccaagcacag tcaaggtctc 480
taggacatgg ccacccctca cctgtggaag cggtcctgct ggggtgggtg ggtgttagtt 540
ggttctggtt tgggtcagag acacccagtg gcccaggtgg gcgtggggcc agggcgcaga 600
cgagaagggg cacgagggct ccgctccgag gacccagcgg caagcaccgg tcccgggcgc 660
gccccagccc acccactcgc gtgcccacgg cggcattatt ccctataagg atctgaacga 720
tccgggggcg gccccgcccc gttacccctt gcccccggcc ccgccccctt tttggagggc 780
cgatgaggta atgcggctct gccattggtc tgagggggcg ggccccaaca gcccgaggcg 840
gggtccccgg gggcccagcg ctatatcact cggccgccca ggcagcggcg cagagcgggc 900
agcaggcagg cggcgggcgc tcagacggct tctcctcctc ctcttgctcc tccagctcct 960
gctccttcgc cgggaggccg cccgccgagt cctgcgccag cgccgaggca gcctcgctgc 1020
gccccatccc gtcccgccgg gcactcggag ggcagcgcgc cggaggccaa ggttgccccg 1080
cacggcccgg cgggcgagcg agctcgggct gcagcagccc cgccggcggc gcgcacggca 1140
actttggaga ggcgagcagc agccccggca gcggcggcag cagcggcaat gaccccttgg 1200
ctcgggctca tcgtgctcct gggcagctgg agcctggggg actggggcgc cgaggcgtgc 1260
acatgctcgc ccagccaccc ccaggacgcc ttctgcaact ccgacatcgt gatccgggcc 1320
aaggtggtgg ggaagaagct ggtaaaggag gggcccttcg gcacgctggt ctacaccatc 1380
aagcagatga agatgtaccg aggcttcacc aagatgcccc atgtgcagta catccacacg 1440
gaagcttccg agagtctctg tggccttaag ctggaggtca acaagtacca gtacctgctg 1500
acaggtcgcg tctatgatgg caagatgtac acggggctgt gcaacttcgt ggagaggtgg 1560
gaccagctca ccctctccca gcgcaagggg ctgaactatc ggtatcacct gggttgtaac 1620
tgcaagatca agtcctgcta ctacctgcct tgctttgtga cttccaagaa cgagtgtctc 1680
tggaccgaca tgctctccaa tttcggttac cctggctacc agtccaaaca ctacgcctgc 1740
atccggcaga agggcggcta ctgcagctgg taccgaggat gggccccccc ggataaaagc 1800
atcatcaatg ccacagaccc ctgagcgcca gaccctgccc cacctcactt ccctcccttc 1860
ccgctgagct tcccttggac actaactctt cccagatgat gacaatgaaa ttagtgcctg 1920
ttttcttgca aatttagcac ttggaacatt taaagaaagg tctatgctgt catatggggt 1980
ttattgggaa ctatcctcct ggccccaccc tgccccttct ttttggtttt gacatcattc 2040
atttccacct gggaatttct ggtgccatgc cagaaagaat gaggaacctg tattcctctt 2100

cttcgtgata atataatctc tattttttta ggaaaacaaa aatgaaaaac tactccattt 2160
gaggattgta attcccaccc ctcttgcttc ttccccacct caccatctcc cagaccctct 2220
tccctttgcc cttctcctcc aatacataaa ggacacagac aaggaacttg ctgaaaggcc 2280
aaccatttca ggatcagtca aaggcagcaa gcagatagac tcaaggtgtg tgaaagatgt 2340
tatacaccag gagctgccac tgcatgtccc aaccagactg tgtctgtctg tgtctgcatg 2400
taagagtgag ggagggaagg aaggaactac aagagagtcg gagatgatgc agcacacaca 2460
caattcccca gcccagtgat gcttgtgttg accagatgtt cctgagtctg gagcaagcac 2520
ccaggccaga ataacagagc tttcttagtt ggtgaagact taaacatctg cctgaggtca 2580
ggaggcaatt tgcctgcctt gtacaaaagc tcaggtgaaa gactgagatg aatgtctttc 2640
ctctccctgc ctcccaccag acttcctcct ggaaaacgct ttggtagatt tggccaggag 2700
ctttctttta tgtaaattgg ataaatacac acaccataca ctatccacag atatagccaa 2760
gtagatttgg gtagaggata ctatttccag aatagtgttt agctcaccta gggggatatg 2820
tttgtataca catttgcata tacccacatg gggacataag ctaatttttt tacaggacac 2880
agaattctgt tcaatgctgt taaatatgcc aatagtttaa tctcttctat tttgttgtcg 2940
ttgcttgttt gaagaaaatc atgacattcc aagttgacat ttttttttca ttttaattaa 3000
aatttgaaat tctgaacacc gtcagcaccc tctcttccct atcatgggtc atctgacccc 3060
tgtccgtctc cttgtccctg cttcatgttt gggggccttt ctttaactgc cttcctggct 3120
tagctcagat ggcagatgag agtgtagtca agggcctggg cacaggaggg agagctgcag 3180
agtgtcctgc ctgccttggc tggagggaca cctctcctgg gtgtggagac agcttggttc 3240
cctttcccta gctccctggt gggtgaatgc cacctcctga gatcctcacc tcttggaatt 3300
aaaattgttg gtcactgggg aaagcctgag tttgcaacca gttgtagggt ttctgttgtg 3360
tttttttttt tttttttgaa ataaaactat aatataaatt ctcctattaa ataaaattat 3420
tttaagtttt agtgtcaaaa gtgagatgct gagagtaggt gataatgtat attttacaga 3480
gtgggggttg gcaggatggt gacattgaac atgattgctc tctgtctctt ttttcagctt 3540
atgggtattt atcttctatt agtatttgta tcttcagttc attccacttt aggaaacaga 3600
gctgccaatt gaaacagaag aagaaaaaaa aaaaaagcag cagacaacac actgtagagt 3660
cttgcacaca cacaagtgcc caggcaaggt gcttggcaga accgcagagt gggaagagag 3720
taccggcatc gggtttcctt gggatcaatt tcattaccgt gtacctttcc cattgtggtc 3780
atgccatttg gcagggggag aatgggaggc ttggccttct ttgtgaggca gtgtgagcag 3840
aagctgatgc cagcatgtca ctggttttga agggatgagc ccagacttga tgttttggga 3900
ttgtccttat tttaacctca aggtctcgca tggtggggcc cctgaccaac ctacacaagt 3960
tccctcccac aagtggacat cagtgtcttc tctgtgaggc atctggccat tcgcactccc 4020
tggtgtggtc agcctctctc acacaaggag gaacttgggt gaaggctgag tgtgaggcac 4080
ctgaagtttc cctgcggagt cgataaatta gcagaaccac atccccatct gttaggcctt 4140

ggtgaggagg ccctgggcaa agaagggtct ttcgcaaagc gatgtcagag ggcggttttg 4200
agctttctat aagctatagc tttgtttatt tcacccgttc acttactgta taatttaaaa 4260
tcatttatgt agctgagaca cttctgtatt tcaatcatat catgaacatt ttattttgct 4320
aaatcttgtg tcatgtgtag gctgtaatat gtgtacattg tgtttaagag aaaaatgaaa 4380
cccacatgcc gccattttcc tgaatcaaat tctgcagtgg aatggagagg aaaatacttc 4440
taggcaagca gctagactgg tgaattgggg gaaatagaag gaactagtaa ctgagactcc 4500
tccagcctcc tccctattgg aatcccaatg gctcctggag taggaaaaaa gtttaaacta 4560
cattcatgtt cttgttctgt gtcactcggc cctgggtagt ctaccattta cttcacccca 4620
agtcctgctg cccatccagt tgggaagcca tgattttcct aagaatccag ggccatggga 4680
gatacaattc caagttctcg cttcctcctt tgggcatctc ttctgcctcc caatcaagga 4740
agctccatgc tcaggctctc agctctcggg ccagtgctct gctctgtcca gggtaggtaa 4800
tactgggaga ctcctgtctt ttaccctccc ctcgttccag acctgcctca tggtggcaac 4860
atggttcttg aacaattaaa gaaacaaatg actttttgga atagccctgt ctagggcaaa 4920
ctgtggcccc caggagacac tacccttcca tgccccagac ctctgtcttg catgtgacaa 4980
ttgacaatct ggactacccc aagatggcac ccaagtgttt ggcttctggc tacctaaggt 5040
taacatgtca ctagagtatt tttatgagag acaaacatta taaaaatctg atggcaaaag 5100
caaaacaaaa tggaaagtag gggaggtgga tgtgacaaca acttccaaat tggctctttg 5160
gaggcgagag gaaggggaga acttggagaa tagtttttgc tttgggggta gaggcttctt 5220
agattctccc agcatccgcc tttcccttta gccagtctgc tgtcctgaaa cccagaagtg 5280
atggagagaa accaacaaga gatctcgaac cctgtctaga aggaatgtat ttgttgctaa 5340
atttcgtagc actgtttaca gttttcctcc atgttattta tgaattttat attccgtgaa 5400
tgtatattgt cttgtaatgt tgcataatgt tcacttttta tagtgtgtcc tttattctaa 5460
acagtaaagt ggttttattt ctatcac 5487

<210> 64
<211> 866
<212> DNA
<213> Homo sapiens

<220>
<221> misc_feature
<223> APC >gi|551463|gb|U02509.1|HSU02509 Human adenomatous polyposis coli
(APC) gene, promoter sequence

<220>
<221> misc_feature
<222> (315)..(315)
<223> n is a, c, g, or t

<400> 64
acttatatat ctgacagttg atttgtcctc acctctaaat tggaatttaa gcatcacctg 60

gttcgattta atgcaatgta gaatttgcat taaaatacta cattaaagcc tcagatttgt 120

agtagctaac agcacttcta tgtatgtgtc agggactgct ctaaatactt catatatatt 180

aactcctcta ttctgtactt ctgttcccgt tttatacagc aggaaattga aacactgaga 240

ggttaagtaa ctaaagttac agagctagag tgacaggagt aaagcttcaa ctcaggcaac 300

ccagacgtcc agagntctga tctccactac taagctgcta gcatagcttt tctggtaact 360

atttttaatt caatataatt cgaatgatct atctaacaag tcatcactct gacaactcag 420

tgacttgtaa tgtaaaatta ttcattgtaa ttcacttaat attattgttt ctctgtgctg 480

caaaaatcat agcaatcgag atgtaattta ttactctccc tcccacctcc ggcatcttgt 540

gctaatcctt ctgccctgcg gacctccccc gactctttac tatgcgtgtc aactgccatc 600

aacttccttg cttgctgggg actggggccg tgagggcata cccccgaggg gtacggggct 660

agggctaggc aggctgtgcg gttgggcggg gccctgtgcc ccactgcgga gtgcgggtcg 720

ggaagcggag agagaagcag ctgtgtaatc cgctggatgc ggaccagggc gctccccatt 780

cccgtcggga gcccgccgat tggctgggtg tgggcgcacg tgaccgacat gtggctgtat 840

tggtgcagcc cgccagggtg tcactg 866

<210> 65
<211> 10386
<212> DNA
<213> Homo sapiens


<220>
<221> misc_feature
<223> >gi|21626462|ref|NM_000038.2| Homo sapiens adenomatosis polyposis
coli (APC), mRNA

<220>
<221> misc_feature
<222> (9521)..(9521)
<223> n is a, c, g, or t

<400> 65
attgaggact cggaaatgag gtccaagggt agccaaggat ggctgcagct tcatatgatc 60

agttgttaaa gcaagttgag gcactgaaga tggagaactc aaatcttcga caagagctag 120

aagataattc caatcatctt acaaaactgg aaactgaggc atctaatatg aaggaagtac 180

ttaaacaact acaaggaagt attgaagatg aagctatggc ttcttctgga cagattgatt 240

tattagagcg tcttaaagag cttaacttag atagcagtaa tttccctgga gtaaaactgc 300

ggtcaaaaat gtccctccgt tcttatggaa gccgggaagg atctgtatca agccgttctg 360

gagagtgcag tcctgttcct atgggttcat ttccaagaag agggtttgta aatggaagca 420

gagaaagtac tggatattta gaagaacttg agaaagagag gtcattgctt cttgctgatc 480

ttgacaaaga agaaaaggaa aaagactggt attacgctca acttcagaat ctcactaaaa 540

gaatagatag tcttccttta actgaaaatt tttccttaca aacagatatg accagaaggc 600

aattggaata tgaagcaagg caaatcagag ttgcgatgga agaacaacta ggtacctgcc 660

aggatatgga aaaacgagca cagcgaagaa tagccagaat tcagcaaatc gaaaaggaca 720
tacttcgtat acgacagctt ttacagtccc aagcaacaga agcagagagg tcatctcaga 780
acaagcatga aaccggctca catgatgctg agcggcagaa tgaaggtcaa ggagtgggag 840
aaatcaacat ggcaacttct ggtaatggtc agggttcaac tacacgaatg gaccatgaaa 900
cagccagtgt tttgagttct agtagcacac actctgcacc tcgaaggctg acaagtcatc 960
tgggaaccaa ggtggaaatg gtgtattcat tgttgtcaat gcttggtact catgataagg 1020
atgatatgtc gcgaactttg ctagctatgt ctagctccca agacagctgt atatccatgc 1080
gacagtctgg atgtcttcct ctcctcatcc agcttttaca tggcaatgac aaagactctg 1140
tattgttggg aaattcccgg ggcagtaaag aggctcgggc cagggccagt gcagcactcc 1200
acaacatcat tcactcacag cctgatgaca agagaggcag gcgtgaaatc cgagtccttc 1260
atcttttgga acagatacgc gcttactgtg aaacctgttg ggagtggcag gaagctcatg 1320
aaccaggcat ggaccaggac aaaaatccaa tgccagctcc tgttgaacat cagatctgtc 1380
ctgctgtgtg tgttctaatg aaactttcat ttgatgaaga gcatagacat gcaatgaatg 1440
aactaggggg actacaggcc attgcagaat tattgcaagt ggactgtgaa atgtacgggc 1500
ttactaatga ccactacagt attacactaa gacgatatgc tggaatggct ttgacaaact 1560
tgacttttgg agatgtagcc aacaaggcta cgctatgctc tatgaaaggc tgcatgagag 1620
cacttgtggc ccaactaaaa tctgaaagtg aagacttaca gcaggttatt gcaagtgttt 1680
tgaggaattt gtcttggcga gcagatgtaa atagtaaaaa gacgttgcga gaagttggaa 1740
gtgtgaaagc attgatggaa tgtgctttag aagttaaaaa ggaatcaacc ctcaaaagcg 1800
tattgagtgc cttatggaat ttgtcagcac attgcactga gaataaagct gatatatgtg 1860
ctgtagatgg tgcacttgca tttttggttg gcactcttac ttaccggagc cagacaaaca 1920
ctttagccat tattgaaagt ggaggtggga tattacggaa tgtgtccagc ttgatagcta 1980
caaatgagga ccacaggcaa atcctaagag agaacaactg tctacaaact ttattacaac 2040
acttaaaatc tcatagtttg acaatagtca gtaatgcatg tggaactttg tggaatctct 2100
cagcaagaaa tcctaaagac caggaagcat tatgggacat gggggcagtt agcatgctca 2160
agaacctcat tcattcaaag cacaaaatga ttgctatggg aagtgctgca gctttaagga 2220
atctcatggc aaataggcct gcgaagtaca aggatgccaa tattatgtct cctggctcaa 2280
gcttgccatc tcttcatgtt aggaaacaaa aagccctaga agcagaatta gatgctcagc 2340
acttatcaga aacttttgac aatatagaca atttaagtcc caaggcatct catcgtagta 2400
agcagagaca caagcaaagt ctctatggtg attatgtttt tgacaccaat cgacatgatg 2460
ataataggtc agacaatttt aatactggca acatgactgt cctttcacca tatttgaata 2520
ctacagtgtt acccagctcc tcttcatcaa gaggaagctt agatagttct cgttctgaaa 2580
aagatagaag tttggagaga gaacgcggaa ttggtctagg caactaccat ccagcaacag 2640
aaaatccagg aacttcttca aagcgaggtt tgcagatctc caccactgca gcccagattg 2700

ccaaagtcat ggaagaagtg tcagccattc atacctctca ggaagacaga agttctgggt 2760
ctaccactga attacattgt gtgacagatg agagaaatgc acttagaaga agctctgctg 2820
cccatacaca ttcaaacact tacaatttca ctaagtcgga aaattcaaat aggacatgtt 2880
ctatgcctta tgccaaatta gaatacaaga gatcttcaaa tgatagttta aatagtgtca 2940
gtagtagtga tggttatggt aaaagaggtc aaatgaaacc ctcgattgaa tcctattctg 3000
aagatgatga aagtaagttt tgcagttatg gtcaataccc agccgaccta gcccataaaa 3060
tacatagtgc aaatcatatg gatgataatg atggagaact agatacacca ataaattata 3120
gtcttaaata ttcagatgag cagttgaact ctggaaggca aagtccttca cagaatgaaa 3180
gatgggcaag acccaaacac ataatagaag atgaaataaa acaaagtgag caaagacaat 3240
caaggaatca aagtacaact tatcctgttt atactgagag cactgatgat aaacacctca 3300
agttccaacc acattttgga cagcaggaat gtgtttctcc atacaggtca cggggagcca 3360
atggttcaga aacaaatcga gtgggttcta atcatggaat taatcaaaat gtaagccagt 3420
ctttgtgtca agaagatgac tatgaagatg ataagcctac caattatagt gaacgttact 3480
ctgaagaaga acagcatgaa gaagaagaga gaccaacaaa ttatagcata aaatataatg 3540
aagagaaacg tcatgtggat cagcctattg attatagttt aaaatatgcc acagatattc 3600
cttcatcaca gaaacagtca ttttcattct caaagagttc atctggacaa agcagtaaaa 3660
ccgaacatat gtcttcaagc agtgagaata cgtccacacc ttcatctaat gccaagaggc 3720
agaatcagct ccatccaagt tctgcacaga gtagaagtgg tcagcctcaa aaggctgcca 3780
cttgcaaagt ttcttctatt aaccaagaaa caatacagac ttattgtgta gaagatactc 3840
caatatgttt ttcaagatgt agttcattat catctttgtc atcagctgaa gatgaaatag 3900
gatgtaatca gacgacacag gaagcagatt ctgctaatac cctgcaaata gcagaaataa 3960
aagaaaagat tggaactagg tcagctgaag atcctgtgag cgaagttcca gcagtgtcac 4020
agcaccctag aaccaaatcc agcagactgc agggttctag tttatcttca gaatcagcca 4080
ggcacaaagc tgttgaattt tcttcaggag cgaaatctcc ctccaaaagt ggtgctcaga 4140
cacccaaaag tccacctgaa cactatgttc aggagacccc actcatgttt agcagatgta 4200
cttctgtcag ttcacttgat agttttgaga gtcgttcgat tgccagctcc gttcagagtg 4260
aaccatgcag tggaatggta agtggcatta taagccccag tgatcttcca gatagccctg 4320
gacaaaccat gccaccaagc agaagtaaaa cacctccacc acctcctcaa acagctcaaa 4380
ccaagcgaga agtacctaaa aataaagcac ctactgctga aaagagagag agtggaccta 4440
agcaagctgc agtaaatgct gcagttcaga gggtccaggt tcttccagat gctgatactt 4500
tattacattt tgccacggaa agtactccag atggattttc ttgttcatcc agcctgagtg 4560
ctctgagcct cgatgagcca tttatacaga aagatgtgga attaagaata atgcctccag 4620
ttcaggaaaa tgacaatggg aatgaaacag aatcagagca gcctaaagaa tcaaatgaaa 4680
accaagagaa agaggcagaa aaaactattg attctgaaaa ggacctatta gatgattcag 4740

atgatgatga tattgaaata ctagaagaat gtattatttc tgccatgcca acaaagtcat 4800
cacgtaaagc aaaaaagcca gcccagactg cttcaaaatt acctccacct gtggcaagga 4860
aaccaagtca gctgcctgtg tacaaacttc taccatcaca aaacaggttg caaccccaaa 4920
agcatgttag ttttacaccg ggggatgata tgccacgggt gtattgtgtt gaagggacac 4980
ctataaactt ttccacagct acatctctaa gtgatctaac aatcgaatcc cctccaaatg 5040
agttagctgc tggagaagga gttagaggag gagcacagtc aggtgaattt gaaaaacgag 5100
ataccattcc tacagaaggc agaagtacag atgaggctca aggaggaaaa acctcatctg 5160
taaccatacc tgaattggat gacaataaag cagaggaagg tgatattctt gcagaatgca 5220
ttaattctgc tatgcccaaa gggaaaagtc acaagccttt ccgtgtgaaa aagataatgg 5280
accaggtcca gcaagcatct gcgtcgtctt ctgcacccaa caaaaatcag ttagatggta 5340
agaaaaagaa accaacttca ccagtaaaac ctataccaca aaatactgaa tataggacac 5400
gtgtaagaaa aaatgcagac tcaaaaaata atttaaatgc tgagagagtt ttctcagaca 5460
acaaagattc aaagaaacag aatttgaaaa ataattccaa ggacttcaat gataagctcc 5520
caaataatga agatagagtc agaggaagtt ttgcttttga ttcacctcat cattacacgc 5580
ctattgaagg aactccttac tgtttttcac gaaatgattc tttgagttct ctagattttg 5640
atgatgatga tgttgacctt tccagggaaa aggctgaatt aagaaaggca aaagaaaata 5700
aggaatcaga ggctaaagtt accagccaca cagaactaac ctccaaccaa caatcagcta 5760
ataagacaca agctattgca aagcagccaa taaatcgagg tcagcctaaa cccatacttc 5820
agaaacaatc cacttttccc cagtcatcca aagacatacc agacagaggg gcagcaactg 5880
atgaaaagtt acagaatttt gctattgaaa atactccagt ttgcttttct cataattcct 5940
ctctgagttc tctcagtgac attgaccaag aaaacaacaa taaagaaaat gaacctatca 6000
aagagactga gccccctgac tcacagggag aaccaagtaa acctcaagca tcaggctatg 6060
ctcctaaatc atttcatgtt gaagataccc cagtttgttt ctcaagaaac agttctctca 6120
gttctcttag tattgactct gaagatgacc tgttgcagga atgtataagc tccgcaatgc 6180
caaaaaagaa aaagccttca agactcaagg gtgataatga aaaacatagt cccagaaata 6240
tgggtggcat attaggtgaa gatctgacac ttgatttgaa agatatacag agaccagatt 6300
cagaacatgg tctatcccct gattcagaaa attttgattg gaaagctatt caggaaggtg 6360
caaattccat agtaagtagt ttacatcaag ctgctgctgc tgcatgttta tctagacaag 6420
cttcgtctga ttcagattcc atcctttccc tgaaatcagg aatctctctg ggatcaccat 6480
ttcatcttac acctgatcaa gaagaaaaac cctttacaag taataaaggc ccacgaattc 6540
taaaaccagg ggagaaaagt acattggaaa ctaaaaagat agaatctgaa agtaaaggaa 6600
tcaaaggagg aaaaaaagtt tataaaagtt tgattactgg aaaagttcga tctaattcag 6660
aaatttcagg ccaaatgaaa cagccccttc aagcaaacat gccttcaatc tctcgaggca 6720
ggacaatgat tcatattcca ggagttcgaa atagctcctc aagtacaagt cctgtttcta 6780

aaaaaggccc accccttaag actccagcct ccaaaagccc tagtgaaggt caaacagcca 6840
ccacttctcc tagaggagcc aagccatctg tgaaatcaga attaagccct gttgccaggc 6900
agacatccca aataggtggg tcaagtaaag caccttctag atcaggatct agagattcga 6960
ccccttcaag acctgcccag caaccattaa gtagacctat acagtctcct ggccgaaact 7020
caatttcccc tggtagaaat ggaataagtc ctcctaacaa attatctcaa cttccaagga 7080
catcatcccc tagtactgct tcaactaagt cctcaggttc tggaaaaatg tcatatacat 7140
ctccaggtag acagatgagc caacagaacc ttaccaaaca aacaggttta tccaagaatg 7200
ccagtagtat tccaagaagt gagtctgcct ccaaaggact aaatcagatg aataatggta 7260
atggagccaa taaaaaggta gaactttcta gaatgtcttc aactaaatca agtggaagtg 7320
aatctgatag atcagaaaga cctgtattag tacgccagtc aactttcatc aaagaagctc 7380
caagcccaac cttaagaaga aaattggagg aatctgcttc atttgaatct ctttctccat 7440
catctagacc agcttctccc actaggtccc aggcacaaac tccagtttta agtccttccc 7500
ttcctgatat gtctctatcc acacattcgt ctgttcaggc tggtggatgg cgaaaactcc 7560
cacctaatct cagtcccact atagagtata atgatggaag accagcaaag cgccatgata 7620
ttgcacggtc tcattctgaa agtccttcta gacttccaat caataggtca ggaacctgga 7680
aacgtgagca cagcaaacat tcatcatccc ttcctcgagt aagcacttgg agaagaactg 7740
gaagttcatc ttcaattctt tctgcttcat cagaatccag tgaaaaagca aaaagtgagg 7800
atgaaaaaca tgtgaactct atttcaggaa ccaaacaaag taaagaaaac caagtatccg 7860
caaaaggaac atggagaaaa ataaaagaaa atgaattttc tcccacaaat agtacttctc 7920
agaccgtttc ctcaggtgct acaaatggtg ctgaatcaaa gactctaatt tatcaaatgg 7980
cacctgctgt ttctaaaaca gaggatgttt gggtgagaat tgaggactgt cccattaaca 8040
atcctagatc tggaagatct cccacaggta atactccccc ggtgattgac agtgtttcag 8100
aaaaggcaaa tccaaacatt aaagattcaa aagataatca ggcaaaacaa aatgtgggta 8160
atggcagtgt tcccatgcgt accgtgggtt tggaaaatcg cctgaactcc tttattcagg 8220
tggatgcccc tgaccaaaaa ggaactgaga taaaaccagg acaaaataat cctgtccctg 8280
tatcagagac taatgaaagt tctatagtgg aacgtacccc attcagttct agcagctcaa 8340
gcaaacacag ttcacctagt gggactgttg ctgccagagt gactcctttt aattacaacc 8400
caagccctag gaaaagcagc gcagatagca cttcagctcg gccatctcag atcccaactc 8460
cagtgaataa caacacaaag aagcgagatt ccaaaactga cagcacagaa tccagtggaa 8520
cccaaagtcc taagcgccat tctgggtctt accttgtgac atctgtttaa aagagaggaa 8580
gaatgaaact aagaaaattc tatgttaatt acaactgcta tatagacatt ttgtttcaaa 8640
tgaaacttta aaagactgaa aaattttgta aataggtttg attcttgtta gagggttttt 8700
gttctggaag ccatatttga tagtatactt tgtcttcact ggtcttattt tgggaggcac 8760
tcttgatggt taggaaaaaa atagtaaagc caagtatgtt tgtacagtat gttttacatg 8820

tatttaaagt agcatcccat cccaacttcc tttaattatt gcttgtctta aaataatgaa 8880
cactacagat agaaaatatg atatattgct gttatcaatc atttctagat tataaactga 8940
ctaaacttac atcagggaaa aattggtatt tatgcaaaaa aaaatgtttt tgtccttgtg 9000
agtccatcta acatcataat taatcatgtg gctgtgaaat tcacagtaat atggttcccg 9060
atgaacaagc tttacccagc ctgtttgctt tactgcatga atgaaactga tggttcaatt 9120
tcagaagtaa tgattaacag ttatgtggtc acatgatgtg catagagata gctacagtgt 9180
aataatttac actattttgt gctccaaaca aaacaaaaat ctgtgtaact gtaaaacatt 9240
gaatgaaact attttacctg aactagattt tatctgaaag taggtagaat ttttgctatg 9300
ctgtaatttg ttgtatattc tggtatttga ggtgagatgg ctgctctttt attaatgaga 9360
catgaattgt gtctcaacag aaactaaatg aacatttcag aataaattat tgctgtatgt 9420
aaactgttac tgaaattggt atttgtttga agggtcttgt ttcacatttg tattaataat 9480
tgtttaaaat gcctctttta aaagcttata taaatttttt ncttcagctt ctatgcatta 9540
agagtaaaat tcctcttact gtaataaaaa caattgaaga agactgttgc cacttaacca 9600
ttccatgcgt tggcacttat ctattcctga aattctttta tgtgattagc tcatcttgat 9660
ttttaacatt tttccactta aacttttttt tcttactcca ctggagctca gtaaaagtaa 9720
attcatgtaa tagcaatgca agcagcctag cacagactaa gcattgagca taataggccc 9780
acataatttc ctctttctta atattataga aattctgtac ttgaaattga ttcttagaca 9840
ttgcagtctc ttcgaggctt tacagtgtaa actgtcttgc cccttcatct tcttgttgca 9900
actgggtctg acatgaacac tttttatcac cctgtatgtt agggcaagat ctcagcagtg 9960
aagtataatc agcactttgc catgctcaga aaattcaaat cacatggaac tttagaggta 10020
gatttaatac gattaagata ttcagaagta tattttagaa tccctgcctg ttaaggaaac 10080
tttatttgtg gtaggtacag ttctggggta catgttaagt gtccccttat acagtggagg 10140
gaagtcttcc ttcctgaagg aaaataaact gacacttatt aactaagata atttacttaa 10200
tatatcttcc ctgatttgtt ttaaaagatc agagggtgac tgatgataca tgcatacata 10260
tttgttgaat aaatgaaaat ttatttttag tgataagatt catacactct gtatttgggg 10320
agagaaaacc tttttaagca tggtggggca ctcagatagg agtgaataca cctacctggt 10380
ggtcat 10386

<210> 66
<211> 7273
<212> DNA
<213> Homo sapiens


<220>
<221> misc_feature
<223> PTGS2 >gi|3282785|gb|AF044206.1| Homo sapiens cyclooxygenase
(COX-2)
gene, promoter and exon 1

<400> 66

ggtacccagg ctggagtgca ctggtgtgat catagctcac taacctcgaa ctcctgggct 60
taggcaatcc tcttgccttg gcctcccaaa gtgccaggat tacaggcatg agccaccaca 120
gtggagctct caattctgat actaataatt tgtgtcttct ctttttttcc ttagcctgac 180
tagagtaatt aactttatgt cttttaaaag aaccaccttt ttggttttac ccattttctt 240
ttttgatttt ctgtttttga tttgattgat atctactcta attttttatt atttcttttc 300
ctctgcttac tttgaattta attacttttc ttttttgtag tctcctaaaa tagaagctta 360
tattattgat tttagatctt tcttcttttc tattacagca ctcaatgcta taaatttccc 420
tctaagcatt gctttcactg catcctacaa tatttcaact ctattgttat ttagctcaaa 480
agaggttctt aatttctatt gggatttctc tttgacccat gtgttattca gaagtgttcc 540
gtgtgatctc caaatatttg ggagtttttc agctatcttt ctattaatca tttcttgttt 600
aattctattg tggcctgaga gcatatattg tatgatttat attcttgtaa atgtgttaag 660
gtgtgtctta tggtgcagaa tgcggtttat cttgctatat gttccttaga gaataatgta 720
tgttctgctg ttattggata aagtagtcta tagatgtcag ttacatctcg ttgattaatg 780
gtgctgttga gttcagctat gtcctaaatg attttctgtc tgctgtatct gtctatttct 840
gacacaaggc tgttgaagtc tccaaccata ataatgaatt aatctatttt tctttgcagt 900
tttatcaatt ttgtcttata tatattgatg ctccattgtt tggcacatac acattaagaa 960
ttgttatgtc ttcttggaga atttaccttt ccataacatg taacatttcc ctttattcct 1020
gataattttt cttgctcaaa agtttgccct gttggaaatt accagaacta ctctggcttt 1080
atttgattag tgttagcatg ctctctcttt ctctattctt acacttttaa tgtatacttg 1140
actttgtatt taaagtgggg ttcttataga aaacatatac ttggtagggt gggaagtaaa 1200
ataaaaagaa atacttgggt attggtttga tccactctaa caatctctat gttttaattg 1260
atgtatttag accattgata cttatttttt tatcctcatc cctgtgatta cccagagagc 1320
tgcttaaatt gattattgat atagacaaat taataattaa tatctaccgt ttgttactgt 1380
tttctatttt tcattgccct tactttctgc tcctattttt tgctcctttt tctgttaatt 1440
taggttttga gttattttat atcattctat tttctctccc ttctcagcat atgaattatc 1500
tttctttttg acttttttag tggctgccct gaaggttgca atgtacattt acaaccagtc 1560
ccaatctcct ttcaaaaaac acaatactgt ttcatggcta gtgcaagtac ctaataataa 1620
gaagtcactc ctaatttctt tctctcattc tttgtatctt tactgttatt catttcactt 1680
gtacataagc tgtaatcttt caatacatta ttgctattat tatttcaaaa catgttatct 1740
attatatcta tttaaaataa gaaaaatagg ccaggtgcag tggcttactc atgtaatccc 1800
agcactttgg gagaccgatg gattgctaga gctcaggaat tcgagaccag cctgggcaac 1860
atagtgaaac cctgtctcta ctaaaaatac aaaaaaaaaa attgctgggc atggtggcat 1920
gggcctgtgg tcacagctac tcgggaggct gaggtgagag gattgcttga gcctgggagg 1980
cagaggttgc agtgaaccaa aatcaagcta ctgcactcca gcctaagtga cagagtgaga 2040

ccctgtctca aaaaaaaaat gaaagaatta tttttattta tcttcactta tttcttctct 2100
aatgctcttt gtttctttag tatgtagatc caagtttcta acctgtatca tttttcttat 2160
ctcaataact tcttttaaca tttctcacaa agcagatcta ctggccacag aatgcctcaa 2220
ttttcatttg tctgagaaaa ccttatttct ccttcacttt tgaaagataa ttttgtaggg 2280
tacagaattc taggttgtag gttttttccc ctcaaagtga aatatttcat tccactcttt 2340
tcttctttgt atggtatctg agaagaagtc agatgtaatt cttatcatta ttacttaaaa 2400
gattgcttct gttcctttct ctcttctcct tcccttcttt ccttctctgt atattacacc 2460
ttttatagtt gccccatatt tcttagatat tatgttttgg ttttcttctg tgtttttttc 2520
tttgattctc agttttagaa gtctctattt atatatctgc aatcgcaggg attctttcct 2580
ctgccatgtc cagtctacta ataagccctt acagacattg ttgacttctg ttccagtgtt 2640
tttgatctct agcatttctc tgattatttc ttggaattgc catctgtcta cttacattac 2700
caacctattc ttgtgtgttg tcttatcata gtaattgcag ttgttttaat ttcataggta 2760
ttgtaatttc aacatctcta ccatatttga cattgattct gatgcttgct ctgtcttatc 2820
aagctatgtt tttgtctttt agtgtgactt ctaatttttt gttgaaagcc aggcatgatg 2880
tactgagtga aagaaactca atacattgta atgtgacgat aagagttcag gggaagtgaa 2940
gcattctata gtcctatagc aggtctcggc cttttagtga gcctgtgcct atgaacggtg 3000
actttcaaca agtgcttttc attccactct tttcctgtcc ttaagtggga caagatcact 3060
gggggggggc tagaattggg tatttccctt ctccaatgta gaagctaaag agagggctgg 3120
agttgggtat ttttcttccc ctgtatggaa agctagaggc agttaaattt ggatattttc 3180
cttcttctaa ttcagttagg ctgcgacaaa aatcccgaca gtttaggctc taatattata 3240
aaataatttc tcttgagtat aggccttatt aagaacacta tactctgatg gagctgaggg 3300
ggagttttct ctgatattca ctgcgagaac ctcgtagagc tccaggaagc aaaactcaca 3360
aaagtgtggg agtcttccag aatttttcct ttgcagactt atctgcactg aacctccaga 3420
aattcatcaa ttacagttca ggttttccta cccaggtact ggttttcatg gaggtttctg 3480
cctgtgcatt tctgctccag taagttgttc ttcttgtatg gtctgtcttt caaatttttt 3540
aagtagggtt atgacctgtc gcctcacttc tctgacagtt ctgagagtgt tgatttttca 3600
gtttgcttag atttttactt gtttttagga tgaagtgaca atttccaagc tcctccctga 3660
catgccagat cagaaactga aagtcctaag cctcatattc tgtgcgtggg tatgttcaca 3720
tcctgcctgc tccagtgccc ccacctcaca ctctctttcc cttccttgtc cccttgtgag 3780
atttctaggt ccaatacaaa gactgtgttc aactcattca actacttggc tcatctgagt 3840
attataatga acaatcacaa aaaaaaatga agtaaaagaa aaatccatca aagaattgag 3900
atatttgaga aaaagaaagg agatcagtgt tttataaaac ttagaaatag attttttaag 3960
tgtttcttca ttgacttatg tgaaaggact tttcttaatt taacaaatta tgtgctttcg 4020
tttatagcct caaaacttct tgtgtagcta agaatgggta aataatcagg ctttactaaa 4080

ggactaacgt aaagatcttc tgtaagtaac atttctgcta ctcaaggaag agataaactt 4140
catggcataa ccttgccaaa gtatactaag aataaccctg acacaaagct cttttttcag 4200
ccaacatgcc atgaaagaaa gaagacaagg ggtgatctcc actctctaag tgaaccacta 4260
aacccaccaa agaagaaacg agggaaatag aaagaggacc cttgcctgag ataatggatc 4320
tgtatgtatg agtagtagaa ccctgctcaa agtacaagga agggaaaaaa aagttagttt 4380
atttggaatt ttggacatta agagtcttta ttgttcattt tcttttaact cacatgaatg 4440
gcttatcact tcaattaata aatatttcat ttcttttcaa tctatattca tgaaacaaat 4500
ctgaaatgaa cagtgcaaca tgtgaatgtt tagaacatta taaaattaaa cacaaaatct 4560
gtctggcaat cttcctagca tcttaggaaa aaagttgaca aaatttcaag cagcagaagg 4620
gggcagtaaa actcaacaga aagctctgga agatttttaa gattcttcct tattttcttt 4680
tcatgtagag tatttcccaa caaatttcag acgctaatag aaattttgta caacagatcc 4740
atatatttgc ctaaaataga cacagaaaca ttgatatatg caaacatgag agctataagt 4800
tttacatgat caaaaccttt tttttatggt acacaatagt cacagtactt ttccatataa 4860
aacaggttta gtggtcttaa tttagtttgg cacatttaat acactcccat gaccagcatc 4920
ccaaatgtac ctatccgttt tattttattg tctcagaatt gtcagttatt taataaatta 4980
tgtaactttt ttccttatgc tcagatttgc acttctttct aaaactctgc ccatccttaa 5040
agtcccagat tctccttgaa cttttttttt tgactttcca agtacatgga actcttcact 5100
ctatcctgct atataagtga cagaatttcc actatgggat agatggagtt caattccttt 5160
gagtttaaaa taatctaaat ataattattc cttatgccct gtttttccct cacttttgta 5220
tccaaatctc ttttcagaca acagaacaat taatgtctga taaggaagac aatgatgatg 5280
atcacttcaa aataagcttg aattcaggat tgtaatgtaa aattttagta ctctctcaca 5340
gtatggattc taacatggct tctaacccaa actaacatta gtagctctaa ctataaactt 5400
caaatttcag tagatgcaac ctactccttt aaaatgaaac agaagattga aattattaaa 5460
ttatcaaaaa gaaaatgatc cacgctctta gttgaaattt catgtaagat tccatgcaat 5520
aaataggagt gccataaatg gaatgatgaa atatgactag aggaggagaa aggcttccta 5580
gatgagatgg aattttagtc atccgtgtct catgaagaat cagatgtgta cactaagcaa 5640
aacagttaaa aaaaaaacct ccaagtgagt ctcttattta tttttttctt ataagacttc 5700
tacaaattga ggtacctggt gtagttttat ttcaggtttt atgctgtcat tttcctgtaa 5760
tgctaaggac ttaggacata actgaatttt ctattttcca cttcttttct ggtgtgtgtg 5820
tatatatata tgtatatata cacacacaca tatacatata tatattttta gtatctcacc 5880
ctcacatgct cctccctgag cactacccat gatagatgtt aaacaaaagc aaagatgaaa 5940
ttccaactgt taaaatctcc cttccatcta attaattcct catccaacta tgttccaaaa 6000
cgagaataga aaattagccc caataagccc aggcaactga aaagtaaatg ctatgttgta 6060
ctttgatcca tggtcacaac tcataatctt ggaaaagtgg acagaaaaga caaaagagtg 6120

aactttaaaa ctcgaattta ttttaccagt atctcctatg aagggctagt aaccaaaata 6180

atccacgcat cagggagaga aatgccttaa ggcatacgtt ttggacattt agcgtccctg 6240

caaattctgg ccatcgccgc ttcctttgtc catcagaagg caggaaactt tatattggtg 6300

acccgtggag ctcacattaa ctatttacag ggtaactgct taggaccagt attatgagga 6360

gaatttacct ttcccgcctc tctttccaag aaacaaggag ggggtgaagg tacggagaac 6420

agtatttctt ctgttgaaag caacttagct acaaagataa attacagcta tgtacactga 6480

aggtagctat ttcattccac aaaataagag ttttttaaaa agctatgtat gtatgtcctg 6540

catatagagc agatatacag cctattaagc gtcgtcacta aaacataaaa catgtcagcc 6600

tttcttaacc ttactcgccc cagtctgtcc cgacgtgact tcctcgaccc tctaaagacg 6660

tacagaccag acacggcggc ggcggcggga gaggggattc cctgcgcccc cggacctcag 6720

ggccgctcag attcctggag aggaagccaa gtgtccttct gccctccccc ggtatcccat 6780

ccaaggcgat cagtccagaa ctggctctcg gaagcgctcg ggcaaagact gcgaagaaga 6840

aaagacatct ggcggaaacc tgtgcgcctg gggcggtgga actcggggag gagagggagg 6900

gatcagacag gagagtgggg actaccccct ctgctcccaa attggggcag cttcctgggt 6960

ttccgatttt ctcatttccg tgggtaaaaa accctgcccc caccgggctt acgcaatttt 7020

tttaagggga gaggagggaa aaatttgtgg ggggtacgaa aaggcggaaa gaaacagtca 7080

tttcgtcaca tgggcttggt tttcagtctt ataaaaagga aggttctctc ggttagcgac 7140

caattgtcat acgacttgca gtgagcgtca ggagcacgtc caggaactcc tcagcagcgc 7200

ctccttcagc tccacagcca gacgccctca gacagcaaag cctacccccc gcgccgcgcc 7260

ctgcccgaag ctt 7273

<210> 67
<211> 4465
<212> DNA
<213> Homo sapiens

<220>
<221> misc_feature
<223> >gi|4506264|ref|NM_000963.1| Homo sapiens
prostaglandin-endoperoxide synthase 2 (prostaglandin G/H synthase
and cyclooxygenase) (PTGS2), mRNA

<400> 67
caattgtcat acgacttgca gtgagcgtca ggagcacgtc caggaactcc tcagcagcgc 60

ctccttcagc tccacagcca gacgccctca gacagcaaag cctacccccg cgccgcgccc 120

tgcccgccgc tcggatgctc gcccgcgccc tgctgctgtg cgcggtcctg gcgctcagcc 180

atacagcaaa tccttgctgt tcccacccat gtcaaaaccg aggtgtatgt atgagtgtgg 240

gatttgacca gtataagtgc gattgtaccc ggacaggatt ctatggagaa aactgctcaa 300

caccggaatt tttgacaaga ataaaattat ttctgaaacc cactccaaac acagtgcact 360

acatacttac ccacttcaag ggattttgga acgttgtgaa taacattccc ttccttcgaa 420

atgcaattat gagttatgtc ttgacatcca gatcacattt gattgacagt ccaccaactt 480
acaatgctga ctatggctac aaaagctggg aagccttctc taacctctcc tattatacta 540
gagcccttcc tcctgtgcct gatgattgcc cgactccctt gggtgtcaaa ggtaaaaagc 600
agcttcctga ttcaaatgag attgtggaaa aattgcttct aagaagaaag ttcatccctg 660
atccccaggg ctcaaacatg atgtttgcat tctttgccca gcacttcacg catcagtttt 720
tcaagacaga tcataagcga gggccagctt tcaccaacgg gctgggccat ggggtggact 780
taaatcatat ttacggtgaa actctggcta gacagcgtaa actgcgcctt ttcaaggatg 840
gaaaaatgaa atatcagata attgatggag agatgtatcc tcccacagtc aaagatactc 900
aggcagagat gatctaccct cctcaagtcc ctgagcatct acggtttgct gtggggcagg 960
aggtctttgg tctggtgcct ggtctgatga tgtatgccac aatctggctg cgggaacaca 1020
acagagtatg cgatgtgctt aaacaggagc atcctgaatg gggtgatgag cagttgttcc 1080
agacaagcag gctaatactg ataggagaga ctattaagat tgtgattgaa gattatgtgc 1140
aacacttgag tggctatcac ttcaaactga aatttgaccc agaactactt ttcaacaaac 1200
aattccagta ccaaaatcgt attgctgctg aatttaacac cctctatcac tggcatcccc 1260
ttctgcctga cacctttcaa attcatgacc agaaatacaa ctatcaacag tttatctaca 1320
acaactctat attgctggaa catggaatta cccagtttgt tgaatcattc accaggcaaa 1380
ttgctggcag ggttgctggt ggtaggaatg ttccacccgc agtacagaaa gtatcacagg 1440
cttccattga ccagagcagg cagatgaaat accagtcttt taatgagtac cgcaaacgct 1500
ttatgctgaa gccctatgaa tcatttgaag aacttacagg agaaaaggaa atgtctgcag 1560
agttggaagc actctatggt gacatcgatg ctgtggagct gtatcctgcc cttctggtag 1620
aaaagcctcg gccagatgcc atctttggtg aaaccatggt agaagttgga gcaccattct 1680
ccttgaaagg acttatgggt aatgttatat gttctcctgc ctactggaag ccaagcactt 1740
ttggtggaga agtgggtttt caaatcatca acactgcctc aattcagtct ctcatctgca 1800
ataacgtgaa gggctgtccc tttacttcat tcagtgttcc agatccagag ctcattaaaa 1860
cagtcaccat caatgcaagt tcttcccgct ccggactaga tgatatcaat cccacagtac 1920
tactaaaaga acgttcgact gaactgtaga agtctaatga tcatatttat ttatttatat 1980
gaaccatgtc tattaattta attatttaat aatatttata ttaaactcct tatgttactt 2040
aacatcttct gtaacagaag tcagtactcc tgttgcggag aaaggagtca tacttgtgaa 2100
gacttttatg tcactactct aaagattttg ctgttgctgt taagtttgga aaacagtttt 2160
tattctgttt tataaaccag agagaaatga gttttgacgt ctttttactt gaatttcaac 2220
ttatattata agaacgaaag taaagatgtt tgaatactta aacactatca caagatggca 2280
aaatgctgaa agtttttaca ctgtcgatgt ttccaatgca tcttccatga tgcattagaa 2340
gtaactaatg tttgaaattt taaagtactt ttggttattt ttctgtcatc aaacaaaaac 2400
aggtatcagt gcattattaa atgaatattt aaattagaca ttaccagtaa tttcatgtct 2460

actttttaaa atcagcaatg aaacaataat ttgaaatttc taaattcata gggtagaatc 2520
acctgtaaaa gcttgtttga tttcttaaag ttattaaact tgtacatata ccaaaaagaa 2580
gctgtcttgg atttaaatct gtaaaatcag atgaaatttt actacaattg cttgttaaaa 2640
tattttataa gtgatgttcc tttttcacca agagtataaa cctttttagt gtgactgtta 2700
aaacttcctt ttaaatcaaa atgccaaatt tattaaggtg gtggagccac tgcagtgtta 2760
tctcaaaata agaatatttt gttgagatat tccagaattt gtttatatgg ctggtaacat 2820
gtaaaatcta tatcagcaaa agggtctacc tttaaaataa gcaataacaa agaagaaaac 2880
caaattattg ttcaaattta ggtttaaact tttgaagcaa actttttttt atccttgtgc 2940
actgcaggcc tggtactcag attttgctat gaggttaatg aagtaccaag ctgtgcttga 3000
ataacgatat gttttctcag attttctgtt gtacagttta atttagcagt ccatatcaca 3060
ttgcaaaagt agcaatgacc tcataaaata cctcttcaaa atgcttaaat tcatttcaca 3120
cattaatttt atctcagtct tgaagccaat tcagtaggtg cattggaatc aagcctggct 3180
acctgcatgc tgttcctttt cttttcttct tttagccatt ttgctaagag acacagtctt 3240
ctcatcactt cgtttctcct attttgtttt actagtttta agatcagagt tcactttctt 3300
tggactctgc ctatattttc ttacctgaac ttttgcaagt tttcaggtaa acctcagctc 3360
aggactgcta tttagctcct cttaagaaga ttaaaagaga aaaaaaaagg cccttttaaa 3420
aatagtatac acttatttta agtgaaaagc agagaatttt atttatagct aattttagct 3480
atctgtaacc aagatggatg caaagaggct agtgcctcag agagaactgt acggggtttg 3540
tgactggaaa aagttacgtt cccattctaa ttaatgccct ttcttattta aaaacaaaac 3600
caaatgatat ctaagtagtt ctcagcaata ataataatga cgataatact tcttttccac 3660
atctcattgt cactgacatt taatggtact gtatattact taatttattg aagattatta 3720
tttatgtctt attaggacac tatggttata aactgtgttt aagcctacaa tcattgattt 3780
ttttttgtta tgtcacaatc agtatatttt ctttggggtt acctctctga atattatgta 3840
aacaatccaa agaaatgatt gtattaagat ttgtgaataa atttttagaa atctgattgg 3900
catattgaga tatttaaggt tgaatgtttg tccttaggat aggcctatgt gctagcccac 3960
aaagaatatt gtctcattag cctgaatgtg ccataagact gaccttttaa aatgttttga 4020
gggatctgtg gatgcttcgt taatttgttc agccacaatt tattgagaaa atattctgtg 4080
tcaagcactg tgggttttaa tatttttaaa tcaaacgctg attacagata atagtattta 4140
tataaataat tgaaaaaaat tttcttttgg gaagagggag aaaatgaaat aaatatcatt 4200
aaagataact caggagaatc ttctttacaa ttttacgttt agaatgttta aggttaagaa 4260
agaaatagtc aatatgcttg tataaaacac tgttcactgt tttttttaaa aaaaaaactt 4320
gatttgttat taacattgat ctgctgacaa aacctgggaa tttgggttgt gtatgcgaat 4380
gtttcagtgc ctcagacaaa tgtgtattta acttatgtaa aagataagtc tggaaataaa 4440
tgtctgttta tttttgtact attta 4465

<210> 68
<211> 1336
<212> DNA
<213> Homo sapiens

<220>
<221> misc_feature
<223> 14-3-3-Sigma >gi|45238846|ref|NM_006142.3| Homo sapiens stratifin
(SFN), mRNA

<400> 68
   gagagacaca gagtccggca ttggtcccag gcagcagtta gcccgccgcc cgcctgtgtg 60
   tccccagagc catggagaga gccagtctga tccagaaggc caagctggca gagcaggccg 120
   aacgctatga ggacatggca gccttcatga aaggcgccgt ggagaagggc gaggagctct 180
   cctgcgaaga gcgaaacctg ctctcagtag cctataagaa cgtggtgggc ggccagaggg 240
   ctgcctggag ggtgctgtcc agtattgagc agaaaagcaa cgaggagggc tcggaggaga 300
   aggggcccga ggtgcgtgag taccgggaga aggtggagac tgagctccag ggcgtgtgcg 360
   acaccgtgct gggcctgctg gacagccacc tcatcaagga ggccggggac gccgagagcc 420
   gggtcttcta cctgaagatg aagggtgact actaccgcta cctggccgag gtggccaccg 480
   gtgacgacaa gaagcgcatc attgactcag cccggtcagc ctaccaggag gccatggaca 540
   tcagcaagaa ggagatgccg cccaccaacc ccatccgcct gggcctggcc ctgaactttt 600
   ccgtcttcca ctacgagatc gccaacagcc ccgaggaggc catctctctg gccaagacca 660
   ctttcgacga ggccatggct gatctgcaca ccctcagcga ggactcctac aaagacagca 720
   ccctcatcat gcagctgctg cgagacaacc tgacactgtg gacggccgac aacgccgggg 780
   aagagggggg cgaggctccc caggagcccc agagctgagt gttgcccgcc accgccccgc 840
   cctgccccct ccagtccccc accctgccga gaggactagt atggggtggg aggccccacc 900
   cttctcccct aggcgctgtt cttgctccaa agggctccgt ggagagggac tggcagagct 960
   gaggccacct ggggctgggg atcccactct tcttgcagct gttgagcgca cctaaccact 1020
   ggtcatgccc ccacccctgc tctccgcacc cgcttcctcc cgaccccagg accaggctac 1080
   ttctcccctc ctcttgcctc cctcctgccc ctgctgcctc tgatcgtagg aattgaggag 1140
   tgtcccgcct tgtggctgag aactggacag tggcaggggc tggagatggg tgtgtgtgtg 1200
   tgtgtgtgtg tgtgtgtgtg tgtgcgcgcg cgccagtgca agaccgagat tgagggaaag 1260
   catgtctgct gggtgtgacc atgtttcctc tcaataaagt tcccctgtga cactcaaaaa 1320
   aaaaaaaaaa aaaaaa 1336

<210> 69
<211> 1968
<212> DNA
<213> Homo sapiens

<220>
<221> misc_feature

<223>  RASSF1A      >gi|25777678|ref|NM_007182.4| Homo sapiens Ras association
      (RalGDS/AF-6) domain family 1 (RASSF1), transcript variant A,mRNA

<400>  69
tctcctcagc tccttcccgc cgcccagtct ggatcctggg ggaggcgctg aagtcggggc      60

ccgccctgtg gccccgcccg gcccgcgctt gctagcgccc aaagccagcg aagcacgggc     120

ccaaccgggc catgtcgggg gagcctgagc tcattgagct gcgggagctg gcacccgctg     180

ggcgcgctgg gaagggccgc acccggctgg agcgtgccaa cgcgctgcgc atcgcgcggg     240

gcaccgcgtg caaccccaca cggcagctgg tccctggccg tggccaccgc ttccagcccg     300

cggggcccgc cacgcacacg tggtgcgacc tctgtggcga cttcatctgg ggcgtcgtgc     360

gcaaaggcct gcagtgcgcg cattgcaagt tcacctgcca ctaccgctgc cgcgcgctcg     420

tctgcctgga ctgttgcggg ccccgggacc tgggctggga acccgcggtg gagcgggaca     480

cgaacgtgga cgagcctgtg gagtgggaga cacctgacct ttctcaagct gagattgagc     540

agaagatcaa ggagtacaat gcccagatca acagcaacct cttcatgagc ttgaacaagg     600

acggttctta cacaggcttc atcaaggttc agctgaagct ggtgcgccct gtctctgtgc     660

cctccagcaa gaagccaccc tccttgcagg atgcccggcg gggcccagga cggggcacaa     720

gtgtcaggcg ccgcacttcc ttttacctgc ccaaggatgc tgtcaagcac ctgcatgtgc     780

tgtcacgcac aagggcacgt gaagtcattg aggccctgct gcgaaagttc ttggtggtgg     840

atgacccccg caagtttgca ctctttgagc gcgctgagcg tcacggccaa gtgtacttgc     900

ggaagctgtt ggatgatgag cagcccctgc ggctgcggct cctggcaggg cccagtgaca     960

aggccctgag ctttgtcctg aaggaaaatg actctgggga ggtgaactgg gacgccttca    1020

gcatgcctga actacataac ttcctacgta tcctgcagcg ggaggaggag gagcacctcc    1080

gccagatcct gcagaagtac tcctattgcc gccagaagat ccaagaggcc ctgcacgcct    1140

gcccccttgg gtgacctctt gtacccccag gtggaaggca gacagcaggc agcgccaagt    1200

gcgtgccgtg tgagtgtgac agggccagtg gggcctgtgg aatgagtgtg catggaggcc    1260

ctcctgtgct gggggaatga gcccagagaa cagcgaagta gcttgctccc tgtgtccacc    1320

tgtgggtgta gccaggtatg gctctgcacc cctctgccct cattactggg ccttagtggg    1380

ccagggctgc cctgagaagc tgctccaggc ctgcagcagg agtggtgcag acagaagtct    1440

cctcaatttt tgtctcagaa gtgaaaatct tggagaccct gcaaacagaa cagggtcatg    1500

tttgcagggg tgacggccct catctatgag gaaaggtttt ggatcttgaa tgtggtctca    1560

ggatatcctt atcagagcta agggtgggtg ctcagaataa ggcaggcatt gaggaagagt    1620

cttggtttct ctctacagtg ccaactcctc acacaccctg aggtcaggga gtgctggctc    1680

acagtacagc atgtgcctta atgcttcata tgaggaggat gtccctgggc cagggtctgt    1740

gtgaatgtgg gcactggccc aggttcatac cttatttgct aatcaaagcc agggtctctc    1800

cctcaggtgt tttttatgaa gtgcgtgaat gtatgtaatg tgtggtggcc tcagctgaat    1860

gcctcctgtg ggaaaggggg ttggggtgac agtcatcatc agggcctggg gcctgagaga    1920

attggctcaa taaagatttc aagatcctca aaaaaaaaaa aaaaaaaa 1968